# EUROPÄISCHE PATENTSCHRIFT

(11) **EP 1 419 160 B1**
(45) Veröffentlichungstag und Bekanntmachung des Hinweises auf die Patenterteilung: **10.12.2008**
(21) Anmeldenummer: 02767415.9
(22) Anmeldetag: 21.08.2002
(51) Int. Cl.: C07D 487/04, C07D 207/20

(54) **VERFAHREN ZUR HERSTELLUNG VON 6-(4-CHLORPHENYL)-2,2-DIMETHYL-7-PHENYL-2,3-DIHYDRO-1H-PYRROLIZIN-5-YL-ESSIGSÄURE**
METHOD FOR THE PRODUCTION OF 6-(4-CHLOROPHENYL)-2,2-DIMETHYL-7-PHENYL-2,3-DIHYDRO-1H-PYRROLIZIN-5-YLACETIC ACID
PROCEDE DE PRODUCTION DE l'ACIDE 6-(4-CHLOROPHENYL)-2,2-DIMETHYL-7-PHENYL-2,3-DIHYDRO-1H-PYRROLIZINE-5-YL-ACETIQUE

(30) Priorität: 23.08.2001 DE 10141285
(43) Veröffentlichungstag der Anmeldung: 19.05.2004
(73) Patentinhaber: MERCKLE GMBH, 89143 Blaubeuren (DE)
(72) Erfinder: DANNHARDT, Gerd, 55127 Mainz (DE); KAMMERMEIER, Thomas, 89073 Ulm (DE); MERCKLE, Philipp, 89143 Blaubeuren-Weiler (DE); STRIEGEL, Hans-Günter, 89134 Blaustein (DE); LAUFER, Stefan, 89143 Blaubeuren (DE)
(74) Vertreter: Reitstötter - Kinzebach
(86) Internationale Anmeldenummer: PCT/EP2002/009356
(87) Internationale Veröffentlichungsnummer: WO 2003/018583

(56) Entgegenhaltungen:
- LAUFER S ET AL: "SYNTHESIS AND EVALUATION OF A NOVEL SERIES OF PYRROLIZINE DERIVATIVES AS DUAL CYCLOOXYGENASE-1 AND 5-LIPOXYGENASE INHIBITORS" ARCHIV DER PHARMAZIE, VCH VERLAGSGESELLSCHAFT MBH, WEINHEIM, DE, Bd. 330, September 1997 (1997-09), Seiten 307-312, XP000994926 ISSN: 0365-6233 in der Anmeldung erwähnt
- OTTO BAYER: "Methoden der Organischen Chemie (Houben-Weyl) band VII/2c, Ketone, Teil III" 1977 , GEORG THIEME VERLAG , STUTTGART XP002221990 Seite 2519
- DATABASE CROSSFIRE BEILSTEIN [Online] Beilstein Institut zur Foerderung der chemischen Wissenschaften, Frankfurt am Main, DE; XP002221991 & POTEKHIN ET AL.: "Addition of thiiranes to ..." CHEM.HETEROCYCL.COMPD.(ENGL.TRANSL.), Bd. 19, Nr. 6, 1983, Seiten 622-630,
- KRAUS ET AL.: "Selective reduction..." J.ORG.CHEM, Bd. 45, 1980, Seiten 4262-4263, XP002221988
- AHLBRECHT ET AL.: "4-Oxoalkannitrile(2-Cyanoalkyl-ketone) durch..." SYNTHESIS, Bd. 4, Seiten 421-423, XP002221989
- DANNHARDT ET AL ARCH. PHARM. Bd. 321, 1988, Seiten 159 - 162, XP000994858

## Beschreibung

Die vorliegende Erfindung betrifft ein Verfahren zur Herstellung von 6-(4-Chlorphenyl)-2,2-dimethyl-7-phenyl-2,3-dihydro-1H-pyrrolizin-5-yl-essigsäure (ML300) und zur Herstellung von bei diesem Verfahren auftretenden Zwischenprodukten

ML 3000 ist ein vielversprechender Hemmstoff der Cyclooxygenase und 5-Lipoxygenase und eignet sich somit zur Behandlung von Erkrankungen des rheumatischen Formenkreises und zur präventiven Behandlung von allergisch induzierten Erkrankungen, siehe dazu z. B. Drugs of the Future 1995, 20 (10): 1007-1009. In dieser Publikation findet sich auch ein möglicher Weg zur Herstellung. Weitere Herstellungsmöglichkeiten sind beschrieben in EP-A-397175, WO95/32970, WO95/32971, WO95/32972, Archiv der Pharmazie 312, 896-907 (1979); und 321, 159-162 (1988), J.Med. Chem. 1994 (37), 1894-1897, Arch. Pharm. Med. Chem. 330, 307-312 (1997). Bei allen diesen Synthesen erfolgt der Aufbau des Pyrrolizingrundgerüstes nach der in der Formelübersicht dargestellten Methode:

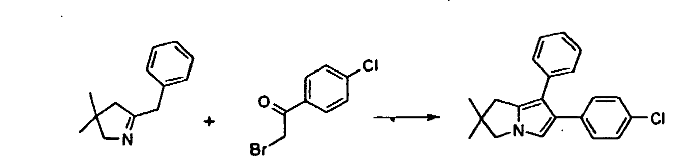

Die Umsetzung wird in Methylenchlorid, Ethanol oder Diethylether durchgeführt. Der bei der Reaktion gebildete Bromwasserstoff wird durch Zusatz wässriger Natriumbicarbonat-Lösung abgefangen.

Die Einführung des Essigsäurerestes in Position 5 kann dann durch Umsetzung mit Diazoessigester, einem Oxalesterchlorid oder Oxalylchlorid und anschließende Verseifung bzw. Verseifung und Reduktion der Ketogruppe mit Hydrazin erfolgen.

Arch. Pharm. 312, 896-907 (1979) beschreibt folgende Umsetzung:

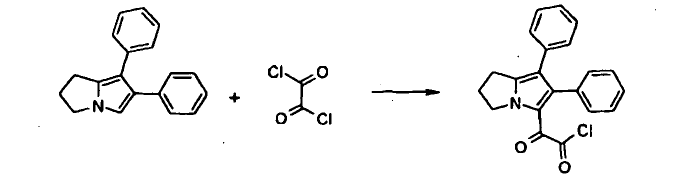

Die Umsetzung wird in Benzol als Lösungsmittel durchgeführt Die COCOCI-Gruppierung wird dann jedoch nicht in die Essigsäure-Gruppe überführt, sondern mit Diethylamin umgesetzt.

Rohes ML 3000, das nach dem Hydrazinverfahren als Kalium-Salz anfällt und das dann aus der mit Mineralsäure sauer gestellten Reaktionsmischung ausgeschieden wird, enthält neben den in Wasser schwerlöslichen Kalium-Salzen auch Hydrazin, Nebenprodukte und Zersetzungsprodukte (Decarboxylierungsprodukt sowie Dimer) als Verunreinigung. Dies erfordert zusätzliche Reinigungsoperationen.

Die Patentanmeldung PCT/EP 01/00852 offenbart ein Verfahren zur Herstellung von ML 3000 durch Umsetzung von 6-(4-Chlorphenyl)-2,2-dimethyl-7-phenyl-2,3-dihydro-1H-pyrrolizin mit Oxalylchlorid und Hydrazin, gefolgt von einem speziellen Aufarbeitungsverfahren. Dabei wird nach der Umsetzung des Pyrrolizins mit Oxalylchlorid das erhaltene Produkt mit Hydrazin und einem Alkalimetallhydroxid in wässriger Phase bei erhöhter Temperatur behandelt, nach beendeter Behandlung durch Zugabe eines mit Wasser nicht oder nur begrenzt mischbaren Ethers ein Drei-Phasen-System erzeugt und ML 3000 durch Ansäuern der mittleren Phase gewonnen. Man erhält ein polymorphes ML 3000 in hoher Ausbeute und reiner, definierter kristalliner Form.

Insgesamt erfolgt die Synthese über die in dem folgenden Formelschema angegebenen Stufen:

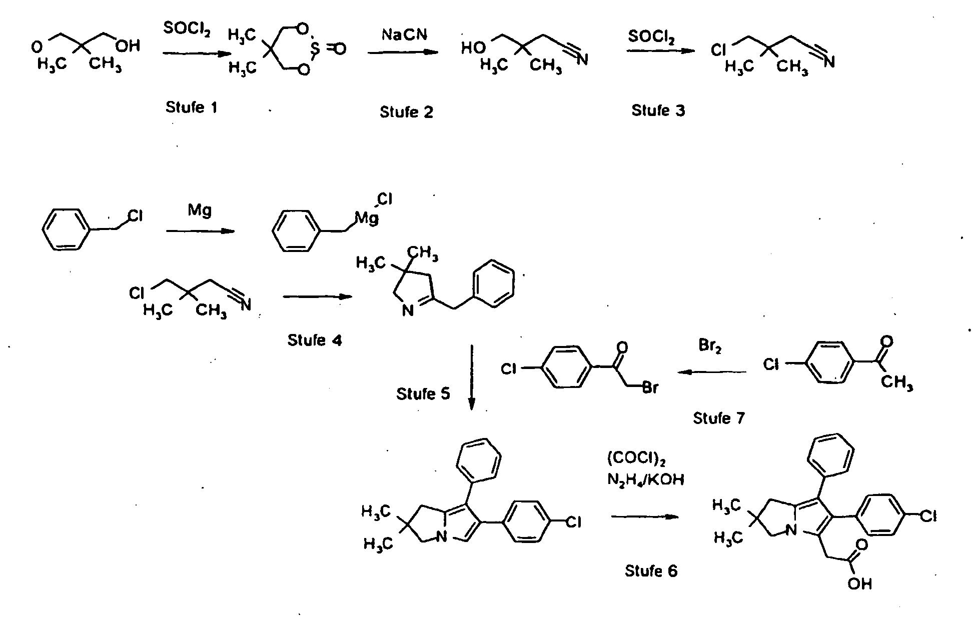

Die Stufen 1 und 2 sind bekannt aus EP 0 172 371 A1. Die Umsetzung von 2,2-Dimethyl-1,3-propandiol mit Thionylchlorid wird in einem inerten organischen Lösungsmittel, z. B. einem halogenierten Kohlenwasserstoff oder einem Ether, bei vorzugsweise 0 bis 60 °C durchgeführt. Die weitere Umsetzung des sich ergebenden 5,5-Dimethyl-1,3,2-dioxathian-2-oxids mit Natriumcyanid zum 4-Hydroxy-3,3-dimethyl-butyronitril erfolgt in DMSO bei etwa 80 bis 120 °C. Stufe 1 liefert Ausbeuten von etwa 93 bis 99 % und Stufe 2 liefert Ausbeuten von etwa 55 bis 60 % mit guter Qualität.

Für Stufe 3, die Umsetzung mit Thionylchlorid zum 4-Chlor-3,3-dimethyl-butyronitril, ist ein Vorprodukt hoher Reinheit erforderlich. Die Rohprodukte aus Stufe 1 und 2 müssen vor der weiteren Umsetzung destilliert werden.

Auch das in Stufe 3 erhaltene 4-Chlor-3,3-dimethyl-butyronitril muss wegen der für die nachfolgende Grignard-Reaktion erforderlichen hohen Reinheit destilliert werden. Bei einer geforderten Reinheit von 97 % sind die Ausbeuten in Stufe 3 unbefriedigend.

Zusätzliche technische Probleme ergeben sich daraus, dass die Rohprodukte aus Stufe 1 und 3 stark sauer aus der Reaktion kommen, was zu Korrosionserscheinungen an den Apparaturen führt.

Weist das 4-Chlor-3,3-dimethyl-butyronitril die erforderliche Reinheit auf, liefern die Addition des Benzylmagnesiumchlorid-Grignardreagenz in Stufe 4 zum 5-Benzyl-3,3-dimethyl-3,4-dihydro-2H-pyrrol und die darauf folgende Zyklisierung mit ω-Brom-4-chloracetophenon in Stufe 5 6-(4-Chlorphenyl)-2,2-dimethyl-7-phenyl-2,3-dihydro-1H-pyrrolizin in guter Qualität und mit einer Ausbeute von 40 bis 45 % über beide Stufen.

Das in Stufe 5 erhaltene Pyrrolizin wird schließlich durch Reaktion mit Oxalylchlorid, gefolgt von Reduktion mit Hydrazin in Anwesenheit eines Alkalimetallhydroxids und Ansäuern, zu 6-(4-Chlorphenyl)-2,2-dimethyl-7-phenyl-2,3-dihydro-1H-pyrrolizin-5-yl-essigsäure (ML 3000) umgesetzt. Die Ausbeute in dieser Stufe 6 beträgt je nach Reinigung des Produkts ca. 62 bis 86 %.

Das bekannte Verfahren liefert ML 3000 mit akzeptabler Reinheit und Ausbeute, weist jedoch einige Nachteile auf, wie die problematische Chemie der zweiten und dritten Stufe, das Erfordernis aufwendiger Reinigung der Zwischenprodukte vor der weiteren Reaktion, insbesondere vor der Grignard-Reaktion, lange Standzeiten, und Korrosionsprobleme an den Apparaturen bei der Reinigung der stark sauren Reaktionsausträge aus Stufe 1 und 3.

Aufgabe der vorliegenden Erfindung ist es daher, ein Verfahren zur Herstellung von 6-(4-Chlorphenyl)-2,2-dimethyl-7-phenyl-2,3-dihydro-1H-pyrrolizin-5-yl-essigsäure (ML3000) bereit zu stellen, bei dem diese Nachteile des Stands der Technik vermieden werden. Mit dem erfindungsgemäßen Verfahren wird insbesondere die Überwindung der technischen Schwierigkeiten in den Stufen I bis 4 der bisherigen Synthese, die Vermeidung der unangenehmen Chemie der zweiten und dritten Reaktionsstufe der Synthese, die Umgehung der Grignard-Reaktion, die Erhöhung der Gesamtausbeuten, die Verringerung der Standzeiten und damit insgesamt eine wirtschaftlichere Gesamtsynthese angestrebt.

Die Aufgabe wird gelöst durch das Verfahren zur Herstellung der Verbindung der Formel I, 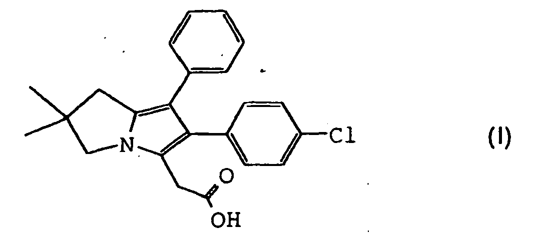 wobei man
a) die Verbindung der Formel IV 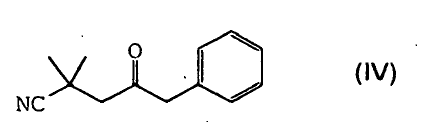 in die Verbindung der Formel III 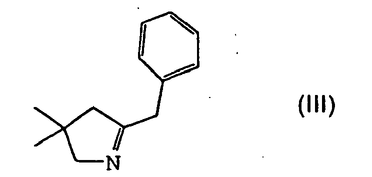 überführt, indem man
   a1) die Verbindung der Formel IV in einer Reinheit von mehr als 90% (m/m) einsetzt und katalytisch mit wasserfreiem Raney-Nickel in Toluol oder einem Gemisch aus Toluol und einem C₁-C₄-Alkohol hydriert, oder
   a2) die Verbindung der Formel IV in das Ketal der Formel IVa 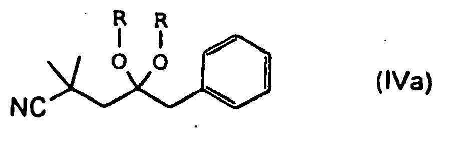 worin die Reste R, die gleich oder verschieden sein können, für C₁-C₄-Alkyl oder zusammen für C₂-C₃-Alkylen stehen, überführt und das Ketal mit wasser-freiem Raney-Nickel bei 5-50 bar Wasserstoffdruck in einem alkoholischen oder aromatischen Lösungsmittel bei einer Temperatur im Bereich von Raumtemperatur bis 70°C katalytisch hydriert,
b) die Verbindung der Formel III mit ω-Brom-4-chloracetophenon zu einer Verbindung der Formel II 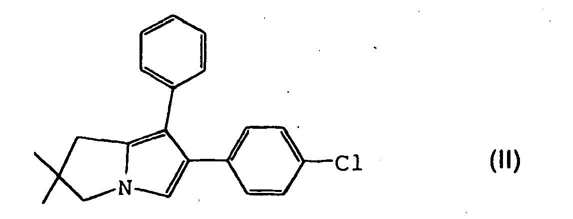 umsetzt und
c) in die Verbindung der Formel II einen Essigsäurerest einführt.

Gegenstand der vorliegenden Erfindung ist auch ein Verfahren zur Herstellung der Verbindung der Formel III durch Hydrieren einer Verbindung der Formel IV und Ringschluss, sowie das entsprechende Verfahren zur Herstellung des Zwischenprodukts der Formel II.

Die Einführung des Essigsäurerestes in die Verbindung der Formel II erfolgt vorzugsweise durch Umsetzung mit Oxalylchlorid und Reduktion der Ketogruppe, bevorzugt mit Hydrazin und einem Alkalimetallhydroxid.

Das bevorzugte erfindungsgemäße Verfahren lässt sich durch folgendes Reaktionsschema veranschaulichen:

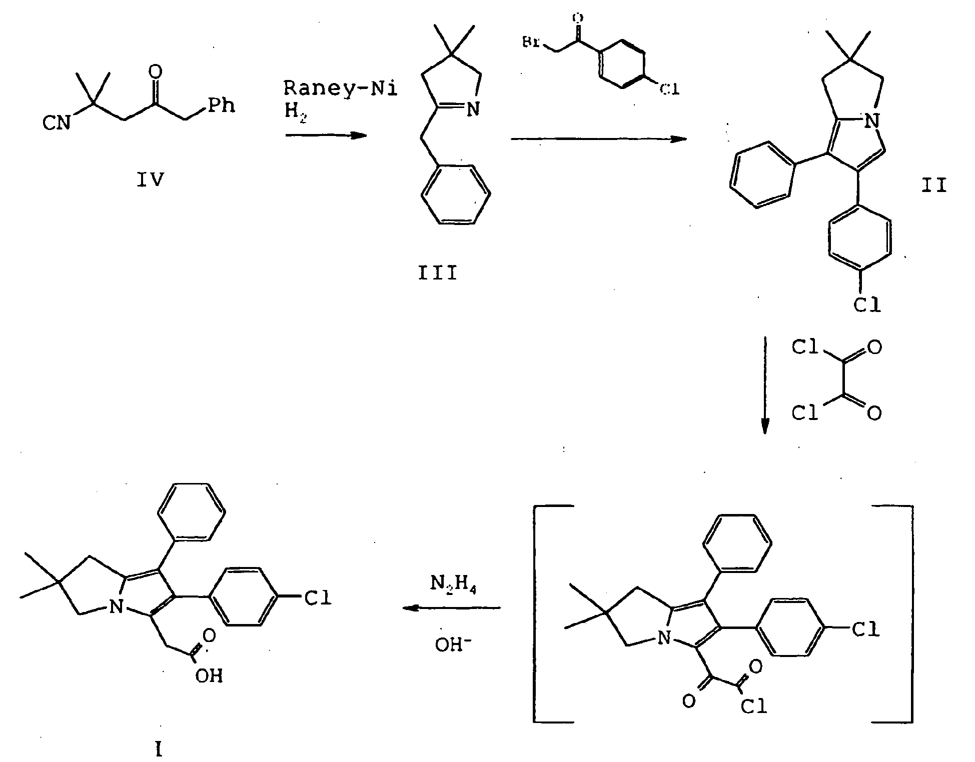

Die Synthese der Verbindung IV erfolgt erfingdungsgemäß bevorzugt über folgende Stufen:
1. Herstellung von 2-(N-Methylanilino)-acrylnitril (V) aus Chloracetaldehyd, N-Methylanilin und einem Alkalimetallcyanid, z.B. Kaliumcyanid. 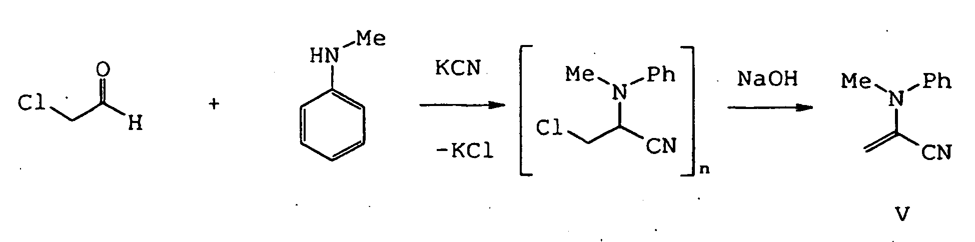
2. Herstellung von 2,2-Dimethyl-4-oxo-5-phenyl-valeronitril (IV) durch Michael-Addition von Isobuttersäurenitril, das mit einer starken Base deprotoniert wurde, an die Verbindung der Formel V, Benzylierung des Michael-Additionsprodukts und Hydrolyse des resultierenden 2-Benzyl-4,4-dimethyl-2-(N-methylanilino)-glutaronitrils.

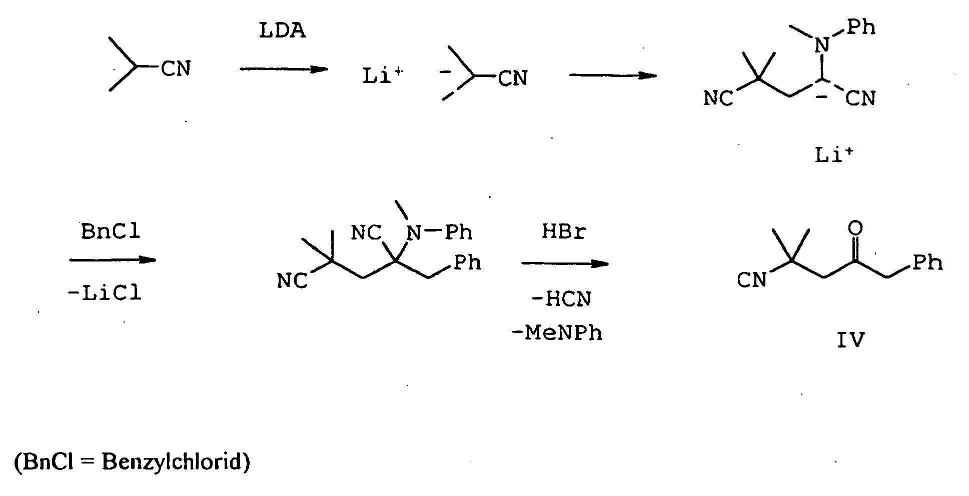

Die Verbindungen der Formel IV und V sowie ihre Herstellung sind bekannt. So wird 2-(N-Methylanilino)-acrylnitril (V) gemäß H. Ahlbrecht und K. Pfaff, Synthesis, 1980, 413, hergestellt. 2,2-Dimethyl-4-oxo-5-phenyl-valeronitril (IV) lässt sich gemäß H. Ahlbrecht und M. Ibe, Synthesis 1985, 421 herstellen.

Das Verfahren zur Herstellung von 2-(N-Methylanilino)-acrylonitril (V) nach der genannten Literaturvorschrift weist jedoch die Nachteile auf, dass weder bei der Umsetzung mit Natrium- oder Kaliumcyanid noch bei der basischen Eliminierung ein vollständiger Umsatz stattfindet, dass Ether zur Extraktion verwendet und das Produkt zur Reinigung destilliert werden muss, wobei starke Verluste durch Zersetzung auftreten. Erfindungsgemäß ist es daher bevorzugt, die Literaturverfahren zu modifizieren und zu verbessern und sie zu einer mehrstufigen Synthese zur Herstellung der Verbindung der Formel III bzw. der Formel II oder I zu kombinieren.

Die erfindungsgemäßen Verbesserungen werden nachfolgend erläutert. Sie können einzeln oder, bevorzugt, als Kombination zur Anwendung kommen. Erfindungsgemäß werden die genannten Nachteile dadurch vermieden, dass die Ausgangsprodukte in anderen Molverhältnissen eingesetzt werden und/oder N-Methylanilin anstelle von Chloracetaldehyd vorgelegt wird und die Reaktionspartner zudosiert werden und/oder die Eliminiening in einem Zwei-Phasen-System Kohlenwasserstoff/Natronlauge unter Zusatz eines Phasentransfer-Katalysators, bevorzugt Benzyltriethylammoniumchlorid, durchgeführt wird. Bevorzugte Verfahrensbedingungen sind nachfolgend angegeben:

Chloracetaldehyd, N-Methylanilin und Kaliumcyanid werden in einem Molverhältnis von 1,1 bis 1,3 :1:1,1 bis 1,3, insbesondere etwa 1,2:1:1,2 eingesetzt. Die Zugabe von N-Methylanilin verläuft exotherm, und Kühlung und/oder Zugabegeschwindigkeit sollten so gewählt werden, dass die Temperatur 25 °C nicht übersteigt. Zu diesem Zweck kann man N-Methylanilin z.B. zu einer Mischung aus Eis und konzentrierter Salzsäure dosieren.

Chloracetaldehyd wird, bevorzugt als wässrige Lösung, zu N-Methylanilinhydrochlorid gegeben, wobei durch geeignete Zugabegeschwindigkeit und ggf. Kühlung eine Temperatur von maximal 20 °C eingehalten wird. Dann wird Kaliumcyanid als wässrige Lösung zudosiert. Durch geeignete Zugabegeschwindigkeit und ggf. Kühlung wird auch hier eine Temperaturobergrenze von 20 °C eingehalten.

Die Zugabe von N-Methylanilin, Chloracetaldehyd und Kaliumcyanid kann auch bei wesentlich tieferen Temperaturen erfolgen, beispielsweise unterhalb 0 °C. Temperaturen nahe der genannten Obergrenzen sind jedoch bevorzugt, da sie eine raschere Zugabe erlauben und einen geringeren Kühlaufwand erfordern, ohne einen negativen Einfluss auf Ausbeute und Qualität des Produkts zu haben.

Wenn der N-Methylanilingehalt der sich bildenden Suspension unterhalb etwa 10 % liegt, wird ein mit Wasser nicht mischbares Lösungsmittel, vorzugsweise ein aliphatischer oder aromatischer Kohlenwasserstoff, insbesondere Toluol zugegeben und das Zwischenprodukt 3-Chlor-2-(N-methylanilino)-propionitril wird in die organische Phase extrahiert. Anschließend wird die Elimination in dem Zwei-Phasen-System Toluol/Natronlauge durchgeführt. Zur Beschleunigung und um eine vollständige Elimination zu erreichen, wird ein Phasentransfer-Katalysator zugesetzt, bevorzugt Benzyltriethylammoniumchlorid. Die Temperatur bei der NaOH-Zugabe sollte 15°C nicht überschreiten, nach beendeter Zugabe kann sie auf Raumtemperatur ansteigen. Die Verwendung von Kaliumhydroxid anstelle von Natriumhydroxid bei der Elimination erschwert die Phasentrennung.

Wenn das Reaktionsgemisch einen Gehalt unter 0,5 % 3-Chlor-2-(N-methylanilino)-propionitril erreicht hat, wird die Produktphase abgetrennt und gegebenenfalls gewaschen, beispielsweise zuerst mit Wasser und dann mit zitronensaurem Wasser. Anschließend wird die organische Phase getrocknet, beispielsweise mit Magnesiumsulfat, und gegebenenfalls filtriert, bevorzugt über ein Druckfilter. Die so erhaltene Lösung von 2-(N-Methylanilino)-acrylnitril in Toluol kann unter Stickstoff bei - 15 °C bis -20 °C problemlos bis zur Weiterverarbeitung gelagert werden. Eine Zersetzung findet bei diesen Temperaturen nicht statt. Das Produkt wird in einer hervorragenden Ausbeute von ca. 95 %, bezogen auf Methylanilin, erhalten.

Die cyanidhaltigen Abwässer und Waschflüssigkeiten sowie der Filterrückstand werden einer Abwasserbehandlung zugeführt.

Das Verfahren zur Herstellung von 2,2-Dimethyl-5-phenyl-4-oxo-valeronitril (IV) nach der genannten Literaturvorschrift arbeitet bis zur Benzylierung des Michael-Additionsprodukts bei -78 °C. Die Benzylierung wird mit kostspieligem Benzylbromid durchgeführt, und die Hydrolyse und Cyanidabspaltung erfolgen in Acetonitril und erfordern eine Reaktionsdauer von etwa 40 bis 50 Stunden. Das Rohprodukt muss destilliert werden.

Bei der erfindungsgemäßen Modifikation des Verfahrens zur Herstellung der Verbindung IV kann auf eine Destillation des Rohprodukts verzichtet werden. Die Reinigung erfolgt ausschließlich durch Umkristallisation. Die Cyanidabspaltung erfolgt im wässrig/organischen System unter Zusatz eines Phasentransfer-Katalysators, wodurch die Reaktionsdauer erheblich verkürzt werden kann. Außerdem ist das erfindungsgemäße Verfahren insofern unkomplizierter, weil es nicht erforderlich ist, die Deprotonierung und Kondensation bei -78 °C durchzuführen. Weiter sind eine Aktivierung mittels des kanzerogenen Hexamethyl-phosphorsäure-triamids (HMPT) und die Verwendung des teuren und aufwendig zu trocknenden Tetrahydrofurans nicht erforderlich. Schließlich kann das preisgünstigere Benzylchlorid anstelle von Benzylbromid verwendet werden. Bevorzugte Verfahrensbedingungen sind nachfolgend angegeben:

Isobutyronitril wird zu einer Lösung einer starken Base in einem inerten Lösungsmittel zudosiert. Als starke Base ist z.B. Natriumamid, Natriumnaphthalenid und bevorzugt Lithiumdiisopropylamid (LDA) geeignet. Die Deprotonierung erfolgt bevorzugt in einem Kohlenwasserstoff, wie Ethylbenzol, als Lösungsmittel und bei Temperaturen unterhalb 10 °C. Dann wird die Verbindung der Formel V, bevorzugt als Lösung in Toluol, zudosiert, wobei die Temperatur ebenfalls bevorzugt unterhalb von 10°C gehalten wird. Die Zulaufgeschwindigkeiten von Isobutyronitril und Verbindung V sind entsprechend zu wählen.

Die bevorzugte Reaktionstemperatur bei der Michael-Addition liegt bei etwa -10 bis -20 °C.

Wenn der Gehalt an Verbindung V in dem Reaktionsgemisch auf unter etwa 2 % gesunken ist, wird Benzylchlorid zudosiert. Vorzugsweise wird die Dosierung bei tiefer Temperatur (etwa -10 bis -20 °C) begonnen, später wird erwärmt, beispielsweise bis auf etwa 50 bis 55 °C.

Wenn der Gehalt an 2,2-Dimethyl-4-(N-methylanilino)-glutarsäuredinitril auf unter etwa 2 % gesunken ist, was mehrere Stunden in Anspruch nimmt, wird die Cyanidabspaltung durchgeführt. Dazu wird das benzylierte Michael-Additionsprodukt im allgemeinen nicht isoliert, sondern es wird durch saure Hydrolyse unter Freisetzung von Cyanwasserstoff und Methyl-anilin unter Rückbildung der Carbonylgruppe in 2,2-Dimethyl-4-oxo-5-phenyl-valeronitril (IV) umgewandelt. Die Hydrolyse wird nach Wasserzugabe bevorzugt unter Phasentransferkatalyse durchgeführt Als Phasentransfer-Katalysator verwendet man bevorzugt Benzyltriethylammoniumchlorid oder Benzyldimethylhexadecylammoniumchlorid. Die Reaktionstemperatur liegt im allgemeinen im Bereich von etwa 20 °C bis etwa 60 °C. Durch die Zugabe des Phasentransfer-Katalysators verkürzt sich die Reaktionszeit auf etwa 15 bis 18 Stunden. Eine weitere Verkürzung der Reaktionszeit kann erzielt werden, wenn man der Toluolphase bis zu etwa 20 Vol.-% Methanol zusetzt und/oder konzentrierte Säure verwendet. Beispielsweise sind dann Reaktionszeiten von nur einer Stunde bei etwa 40 °C möglich.

Zur Cyanidabspaltung/Hydrolyse gibt man eine starke Mineralsäure, wie beispielsweise Bromwasserstoffsäure oder Chlorwasserstoffsäure, zu und lässt das Reaktionsgemisch, bevorzugt bei erhöhter Temperatur, reagieren, bis der Gehalt an benzyliertem Michael-Additionsprodukt auf unter etwa 0,5 % gesunken ist. Dann wird die organische Phase in üblicher Weise aufgearbeitet und das Toluol abdestilliert. Die Temperatur bei der Destillation sollte 50 °C nicht überschreiten. Der Rückstand der Destillation kann dann durch Umkristallisation gereinigt werden oder vor der Umkristallisation einer einmaligen oder mehrmaligen Koevaporation mit Isopropanol unterzogen werden, um Toluolreste zu entfernen.

Das Umkristallisieren kann in Isopropanol erfolgen, aber auch Toluol und Gemische aus Isopropanol und Toluol sind gut geeignete. Bevorzugt wird das Produkt aus zwei Teilen Isopropanol/Toluol im Verhältnis 9:1 umkristallisiert.

Wegen der guten Löslichkeit der Verbindung IV in Isopropanol ist es erforderlich, zum Auskristallisieren abzukühlen, bevorzugt auf Temperaturen von -15°C bis -20 °C.

Das erhaltene Produkt kann unter Umständen auch nach dem Umkristallisieren noch mit Michael-Additionsprodukt verunreinigt sein. Derartige Verunreinigungen sind jedoch nicht störend, da sie bei den weiteren Umsetzungen leicht entfernt werden. Insgesamt hat das Umkristallisieren jedoch einen sehr hohen Reinigungseffekt, so dass das Produkt in sehr reiner Form gewonnen wird.

Als nächste Reaktionsstufe wird das erhaltene 2,2-Dimethyl-4-oxo-5-phenyl-valeronitril (IV) zu 2-Benzyl-4,4-dimethyl-1-pyrrolin (III) katalytisch hydriert. Als Katalysatoren sind Edelmetallkatalysatoren, wie Pt oder Pd, brauchbar. Bevorzugt sind jedoch Raney-Katalysatoren, insbesondere Raney-Ni und Raney-Co.

In Arch. Pharm. 299, 518 (1966) ist die Herstellung von 2-(4-Hydroxyphenyl)-4,4-dihydro-3H-pyrrol durch Hydrierung von 4-Oxo-(4-hydroxyphenyl)butansäurenitril mit Raney-Nickel beschrieben. Die erfindungsgemäße Hydrierung erfolgt, wenn man sie analog zum Literaturverfahren durchführt, d.h. unter Einsatz von wasserhaltigem Raney-Nickel in Alkoholen, jedoch nur schleppend, und bei erhöhtem Druck oder erhöhter Temperatur wird eine deutliche Überhydrierung beobachtet.

Es wurden daher Versuche unternommen die Reaktionsdauer zu verkürzen und die Bildung von Nebenprodukten, insbesondere durch Überhydrierung zum Pyrrolidin, zu verringern. Dabei zeigt sich, dass weder die Erhöhung des Wasserstoffdruckes noch die Erhöhung der Reaktionstemperatur die Reaktionszeit wesentlich verkürzen, sondern dass unter diesen energischeren Bedingungen die Anteile an Nebenprodukten, vor allem an Teilhydrierungsprodukten, an oligomeren Kondensationsprodukten und an überhydriertem Pyrrolidin ansteigen.

Überraschenderweise wurde nun gefunden, dass die Qualität (Reinheit) der eingesetzten Ausgangsverbindung der Formel IV einen starken Einfluss sowohl auf den Zeitverlauf als auch auf die Nebenproduktverteilung der Hydrierung hat. Je reiner das Edukt, desto glatter und problemloser verläuft die Reaktion. Man verwendet die Verbindung IV in einer Reinheit von über 90 %, vorzugsweise von über 95 % (m/m).

Bei der Hydrierung der Nitrilketonverbindung der Formel IV unter Verwendung des Pyrrolins der Formel III wird eine tertiäre Nitrilgruppe in zwei Hydrierteilstufen zur Neopentylamingruppe reduziert, die spontan unter Wasserabspaltung mit der Ketogruppe zur zyklischen Iminogruppe kondensiert. Die zyklische Iminogruppe des Pyrrolin kann zur sekundären zyklischen Aminogruppe im Pyrrolidin weiterhydriert werden. Um dies zu verhindern, verwendet man Raney-Nickel als Katalysator, jedoch nicht, wie üblich, in wasserhaltiger Form, sondern im wesentlichen wasserfrei. Als Lösungsmittel haben sich Toluol und insbesondere Gemische aus Toluol und C₁-C₄-Alkoholen, wie Methanol, Ethanol, Isopropanol, z. B. Toluol/Methanol im Vol.-Verhältnis 8 : 2 bis 6 : 4, als am geeignetsten erwiesen.

Eine weitere Möglichkeit die Überhydrierung zu unterbinden, besteht in der Einführung einer Acetal(Ketal)-Schutzgruppe für die Ketogruppe des Nitrilketons, wobei man die Verbindung der Formel IVa 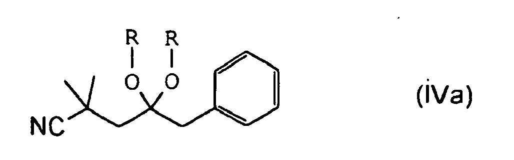 worin die Reste R, die gleich oder verschieden sein können, für einen C₁-C₄-Alkylrest oder zusammen für einen C₂-C₃-Alkylenrest stehen, erhält. Die Hydrierung der tertiären Nitrilgruppe in die Neopentylamingruppe kann so auch unter weniger selektiven Bedingungen geführt werden, wobei man eine Verbindung der Formel IVb 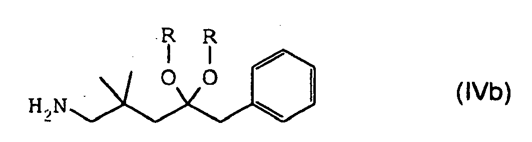 erhält, worin die Reste R die angegebenen Bedeutungen besitzen. Unter den Bedingungen der Ketalspaltung erfolgt in sauren Medien, z. B. in verdünnten Mineralsäuren, gleichzeitig auch die Zyklisierung zum Pyrrolin. Nach Alkalisieren der sauren wässrigen Lösungen der Pyrroliniumsalze erhält man die freie Pyrrolinbase, die mit organischen, mit Wasser nicht mischbaren Lösungsmitteln abgetrennt und nach Entfernen dieser Lösungsmittel in hochreiner Form gewonnen werden kann.

Bevorzugte Reaktionsbedingungen für die direkte Gewinnung der Verbindung III durch Hydrierung der Verbindung der Formel IV werden nachfolgend angegeben:

Wenn ein Gemisch aus Toluol und Methanol als Lösungsmittel verwendet wird, bevorzugt etwa 8 bis 12 Vol.-Teile Toluol/Methanol pro Gew.-Teil der Verbindung V, liegt die Reaktionstemperatur im allgemeinen bei etwa 50-60 °C . Wird in reinem Toluol hydriert, wird die Temperatur etwas niedriger gewählt, beispielsweise 20-30 °C, um eine Überhydrierung zu verhindern. Der Wasserstoff-Druck liegt im allgemeinen bei etwa 4 bis 6 bar.

Vor der Reaktion wird das vorgelegte Raney-Nickel getrocknet, beispielsweise durch ein- oder mehrmaliges Aufschlämmen mit absolutem Methanol oder durch Azeotrop-Destillation.

Wenn die Reaktion vor der Aufnahme der theoretischen Menge Wasserstoff zum Erliegen kommt, kann das Reaktionsgemisch azeotrop destilliert und frisches Lösungsmittel zugesetzt werden. Es kann auch frisches Raney-Nickel zugesetzt und zur Wasserentfernung azeotrop destilliert werden. Die Reaktion dauert im allgemeinen etwa 3 bis 4 Stunden.

Dann lässt man das Raney-Nickel sedimentieren und filtriert die überstehende Reaktionslösung. Der Katalysator kann gegebenenfalls für weitere Hydrierungen verwendet werden. Aus der Reaktionslösung wird das Lösungsmittel abdestilliert. Das Produkt kann über Salzbildung gereinigt werden, z. B. durch Hydrochloridbildung und Freisetzung der Verbindung der Formel IV mit einer Base, beispielsweise Ammoniak, und Rückextraktion.

Alternativ kann auch nur ein Teil des Lösungsmittels abdestilliert werden, z. B. das Methanol bei einem Lösungsmittel-Gemisch aus Toluol/Methanol. In diesem Fall wird der Destillationsrückstand vorteilhafterweise zuerst mit Wasser gewaschen, und nach Abtrennung der Wasserphase kann das Produkt wie oben beschrieben gereinigt werden.

Bei der erfindungsgemäßen Reaktionsführung kann die Hydrierung insbesondere durch Verwendung von wasserfreiem Raney-Nickel als Katalysator und von Toluol oder einem Gemisch aus Toluol und Methanol als Lösungsmittel stark beschleunigt und Nebenreaktionen können in Grenzen gehalten werden.

Die bevorzugten Reaktionsbedingungen für die Gewinnung der Verbindungen der Formel III über die Hydrierung von cyclischen oder acyclischen Acetal(Ketal)-Zwischenstufen werden nachfolgend angegeben:

Das Nitrilketon der Formel IV wird in einem Lösungsmittel, das mit Wasser ein Azeotrop bildet, mit einem Alkohol in Gegenwart eines Säurekatalysators zum Ketal umgewandelt, oder man führt die Umwandlung des Ketons zum Ketal in einem Alkohol in Gegenwart äquivalenter Mengen eines Acetals oder Ketals eines niedrigsiedenden Aldehydes oder Ketones durch. Geeignete Alkohole zur Ketalbildung sind C₁-C₄-Alkanole, wie Methanol, Ethanol oder Glykol, 1,3-Propylenglykol etc. Lösungsmittel, die mit Wasser ein Azeotrop bilden, sind z. B. Toluol, Xylol, Cyclohexan etc.

Eine bevorzugte Ausführungsform ist zum Beispiel die Überführung in ein Oxolanderivat in Toluol mit Ethylenglykol in Anwesenheit einer Säure, wie Toluolsulfonsäure, unter Refluxbedingungen und unter Entfernung des Wassers aus dem Reaktionsgemisch, z. B. mit einem Wasserabscheider. Eine weitere bevorzugte Ausführungsform ist die Überführung in das Dimethylketal in Methanol mit 1,1-Dimethoxyethan in Gegenwart von Pyridinium-Tosylat bei etwa 40 bis 60 °C. Anschließend werden die Ketale durch Waschen mit Alkali behandelt und in Gegenwart eines Hydrierkatalysators hydriert. Erfindungsgemäß wird die Hydrierung des Dioxolanderivates in Gegenwart von wasserfreiem Raney-Nickel bei 5 bis 50 bar Wasserstoffdruck und bei Raumtemperatur bis 70 °C in alkoholischem Lösungsmittel, wie Methanol, oder in einem aromatischen Lösungsmittel, wie Toluol durchgeführt. Die Gewinnung der Verbindung der Formel III erfolgt nach Abfiltrieren des Katalysators durch Ausrühren der als Hydrierungsprodukt anfallenden Aminoketale aus der organischen Phase in eine wässrige verdünnte Mineralsäure. Sowohl die Spaltung der Ketale, als auch die Bildung des zyklischen Imins ist bei RT in der Regel nach 30 min bis 1h vollständig erfolgt. Das zyklische Imin III kann nach Alkalisieren der wässrigen Produktlösung auf pH 9 bis 11 in sehr reiner Form gewonnen werden.

Das 2-Benzyl-4,4-dimethyl-1-pyrrolin der Formel IV wird anschließend mit ω-Brom-4-chloracetophenon zum 6-(4-Chlorphenyl)-2,2-dimethyl-7-phenyl-2,3-dihydro-1H-pyrrolizin der Formel III zyklisiert. Die Reaktion ist aus dem eingangs genannten Stand der Technik bekannt. ω-Brom-4-chloracetophenon kann erhalten werden, wie beispielsweise beschrieben in Bull. Soc. Chim. Fr. 21, 69 (1899).

Die Umsetzung der Verbindung der Formel III mit ω-Brom-4-chloracetophenon erfolgt im allgemeinen in einem polaren organischen Lösungsmittel. Geeignete polare organische Lösemittel sind insbesondere C₁-C₄-Alkohole, wie Methanol, Ethanol, Isopropanol oder Ether, wie Diethylether, Tetrahydrofuran (THF), oder Dioxan. Gemäß der vorliegenden Erfindung ist Methanol als Lösungsmittel besonders bevorzugt. Die Reaktionspartner können in äquimolaren Mengen eingesetzt werden. Es ist jedoch bevorzugt, das ω-Brom-4-chloracetophenon im Überschuss zu verwenden, beispielsweise in einem Überschuss von 10 bis 40 mol%.

Um den bei der Umsetzung frei werdenden Bromwasserstoff abzufangen, arbeitet man in Anwesenheit einer Base. Vorzugsweise verwendet man eine anorganische Base, insbesondere ein Alkalimetallhydrogencarbonat oder Alkalimetallcarbonat, wobei die Natrium- und Kaliumverbindungen besonders bevorzugt sind. Die anorganische Base kann in Form einer wässrigen Lösung eingesetzt werden. Als besonders bevorzugt hat es sich jedoch erwiesen, die anorganische Base in fester Form zu verwenden. Dies erleichtert die Abtrennung der anorganischen Reaktionsprodukte und verringert das Nebenproduktspektrum. Die anorganische Base kann in äquimolaren Mengen eingesetzt werden, bezogen auf die Menge freigesetzten Bromwasserstoffs. Zweckmäßigerweise verwendet man die anorganische Base jedoch im Überschuss, beispielsweise in einem Überschuss von bis zu 1,8 Äquivalenten, bevorzugt etwa 1,4 Äquivalente. Außerdem ist es zweckmäßig, die Umsetzung unter Lichtausschluss vorzunehmen. Die Reaktionstemperatur kann in einem breiten Bereich variiert werden und liegt bevorzugt in einem Bereich von 0 bis 50 °C, besonders bevorzugt bei etwa 18 bis 25 °C. Die Umsetzung ist nach etwa 17 bis 20 Stunden abgeschlossen.

Das erhaltene Rohprodukt der Formel II wird abgetrennt, beispielsweise durch Zentrifugieren, und in üblicher Weise gereinigt, indem die mineralischen Begleitsubstanzen entfernt werden. Dazu wird das Rohprodukt bevorzugt in warmes Wasser, beispielsweise bei 40 bis 45 °C, eingetragen und 1 bis 2 Stunden behandelt. Auf diese Weise wird die Verbindung der Formel II in einer Ausbeute von durchschnittlich 58 % und mit einer Reinheit von mindestens 97 % erhalten. Der Gehalt an dem Isomer mit der 4-Chlorphenylgruppe in 5-Position liegt unter 2 %, der Gehalt an ω-Brom-4-chloracetophenon liegt unter 0, 1 % und der Gehalt an mineralischen Begleitsubstanzen unter 0,5 %.

Zur Herstellung von ML 3000 (1) wird in 5-Position der Verbindung der Formel II eine Essigsäureseitenkette eingeführt. Dies erfolgt vorzugsweise durch Umsetzung der Verbindung der Formel II mit Oxalylchlorid und anschließende Reduktion mit Hydrazin und einem Alkalimetallhydroxid. Die Reaktion ist beispielsweise beschrieben in der WO95/32971, Beispiel 5C, und in PCT/EP 01/00852. Zur Reinigung des Reaktionsprodukts sind unterschiedliche Wege beschrieben. Gemäß WO95/32971 versetzt man das Reaktionsgemisch mit Wasser, säuert an und nimmt die ausgeschiedene Carbonsäure in Diethylether auf. Das Produkt wird gereinigt, indem die ätherische Lösung über einem Trocknungsmittel, wie wasserfreiem Natriumsulfat oder Magnesiumsulfat, einige Zeit gerührt und stehen gelassen, anschließend das mit Wasser gesättigte Sulfat abfiltriert und schließlich der Ether in der Wärme abgedampft wird. Die aus der Mutterlauge beim Konzentrieren kristallisierende Substanz wird gesammelt und getrocknet. Bei diesem Verfahren der Isolierung und Reinigung werden noch im Reinigungsschritt und während der Trocknung einige Zersetzungsprodukte neu gebildet, so dass eine weitere aufwendige Reinigung des ML 3000, z. B. durch Umkristallisation erforderlich ist, um Pharmaqualität zu erhalten.

Bei dem alternativen Reinigungsverfahren wird nach der Reduktion mit Hydrazin und einem Alkalimetallhydroxid zu dem Reaktionsgemisch, gegebenenfalls bei höherer Temperatur, ein Ether und Wasser zugegeben. Vorzugsweise wird ein mit Wasser begrenzt mischbarer Ether, z.B. Diethylether oder Methyl-t-butylether, verwendet. Durch die Zugabe des Ethers kommt es zur Ausbildung eines Drei-Phasen-Systems, wobei die mittlere Phase die Produktphase ist, die im wesentlichen aus dem Salz von ML 3000 mit dem bei der Umsetzung verwendeten Alkalimetallhydroxid besteht. Die oberste Phase ist die Etherphase, in der sich die organischen Verunreinigungen befinden, und die unterste Phase ist eine stark alkalische wässrige Phase, welche die anorganischen Bestandteile enthält.

Die Phasen werden getrennt und die mittlere Phase wird mit einem Gemisch aus Wasser und dem mit Wasser nur begrenzt mischbarem Ether versetzt, anschließend wird mit einer anorganischen oder organischen Säure angesäuert. Das ML 3000 ist dann in der Etherphase gelöst.

Die Gewinnung des ML 3000 aus der Etherphase kann beispielsweise durch Verdampfen des Ethers und Kristallisation des ML 3000 aus Ethylacetat oder Isopropanol erfolgen. Dabei erhält man Solvate mit 1 Molekül Diethylether auf 2 Moleküle ML 3000 bzw. mit 1 Molekül Ethylacetat auf 2 Moleküle ML 3000.

Eine im wesentlichen lösungsmittelfreie Kristallmodifikation des ML 3000 erhält man, wenn man zu der Etherphase mindestens einen höher als der Ether siedenden Kohlenwasserstoff gibt, den Ether ggf. zumindest teilweise abdestilliert und das in fester, kristalliner Form abgeschiedene ML 3000 in üblicher Weise von der Mutterlauge trennt. Als Kohlenwasserstoff ist insbesondere ein geradkettiger oder verzweigter aliphatischer C₆-C₁₂-Kohlenwasserstoff brauchbar, z.B. n-Hexan, n-Heptan, Cyclohexan, Cycloheptan, etc.

Die nachfolgenden Beispiele erläutern die Erfindung, ohne sie zu beschränken.

### Beispiel 1

### A) 2-(N-Methylanilino)acrylnitril (ca. 50%ige Lösung in Toluol)

In einem 250-1-Emaille-Reaktor werden konz. HCl (32 %, 22,33 kg) und Eis (32,6 kg) eingebracht. N-Methylanilin (17,39 kg, 162,2 Mol) wird unter Wasserkühlung zudosiert, ohne die Temperatur über 25 °C steigen zu lassen (30 min.). Die grüngelbe Lösung wird 5-10 min. bei 15-20 °C gerührt. Beginnend bei dieser Temperatur wird eine wässrige Lösung von Chloracetaldehyd (45 %, 34,2 kg, 196, 1 Mol) unter Wasserkühlung so zudosiert, dass die Innentemperatur unter 20 °C gehalten wird (30 min.). Die Reaktionsmischung wird über die Zumischzeit hinaus noch weitere 5-10 min. bei 15-20 °C gerührt und dann bei dieser Temperatur mit einer Lösung von Kaliumcyanid (12,7 kg, 195,1 Mol) in Wasser (19,5 kg) versetzt. Unter Wasserkühlung wird hierbei die Zugabe so gesteuert, dass eine Temperatur von 20 °C nicht überschritten wird (1 h). Bei einer Temperatur von 18-23 °C wird 110-130 min. nachgerührt. Es bildet sich eine dünnflüssige Suspension. Die gaschromatographische Probe zeigt weniger als 10 % Methylanilin-Gehalt an. Dann werden dem Reaktionsgemisch Toluol (25,7 kg) und anschließend unter Rühren konz. Salzsäure (32 %, 9,3 kg) zugegeben und noch 5-10 min. bei RT gerührt.

Die aus der Apparatur austretende Blausäure wird in einem mit Konz. NaOH befüllten Absorber zurückgehalten.

Bei abgeschaltetem Rührer lässt man die Wasserphase (114 kg, Cyanid-Abwasser1) absitzen und überführt sie in einem geschlossen System in einen Tank zur Entsorgung.

Zur blaugefärbten organischen Phase wird Benzyltriethylammoniumchlorid (0,3 kg) gegeben und auf -5 bis 0 °C gekühlt. Wird diese Innentemperatur erreicht, lässt man Natronlauge (30 %, 32,6 kg) so zufließen, dass die Innentemperatur 15 °C nicht überschreitet (30 min.). Nach vollständiger Zugabe lässt man das Reaktionsgemisch auf RT erwärmen und rührt noch weitere 50-70 min.

Die GC-Analyse einer Probe ergibt einen Gehalt unter 0,5 % für die Zwischenstufe 3-Chlor-2-(N-methylanilino)-propionitril. Ist dieser Wert erreicht, wird mit Wasser (40,7 kg) gewaschen: das Wasser wird zugegeben, das Zwei-Phasengemisch 5-10 min. gerührt, dann lässt man die Wasserphase (79 kg, Cyanid-Abwasser 2) absitzen und überführt sie in den Tank (zu Cyanid-Abwasser I).

Die organische Phase wird nochmals mit Wasser (40,7 kg), das mit Zitronensäure (0,81 kg) angesäuert ist, in der gleichen Weise gewaschen.

Diese zitronensaure Wasserphase (45 kg, Cyanid-Abwasser 3) wird mit den anderen Cyanid-Abwässern vereinigt. Die organische Phase wird bei RT (Raumtemperatur) über Magnesiumsulfat (3,8 kg) für 10-20 min. getrocknet. Eine Karl-Fischer Titration ergibt einen Wassergehalt unter 0,2 %. Diese Toluollösung (50-52 kg) wird über ein Druckfilter filtriert und zur Verwendung in der nächsten Stufe abgefüllt. Der Filterrückstand (4,8 kg) wird mit den Cyanid-Abwässern vereinigt. Diese Cyanid-Abwässer werden der Abwasserbehandlung zugeführt. Die bei Raumtemperatur instabile Lösung des 2-(N-Methylanilino)-acrylnitrils (53,86 kg) wird bis zur Weiterverarbeitung bei -15 bis -20 °C unter Stickstoff gelagert. Zur Gehaltsbestimmung wird ein 50 mL Muster gezogen. Von 30 mL dieser Probe wird ein Trocknungsrückstand bestimmt, indem Toluol im Vakuum bei max. 70 °C weitgehend abgedampft wird. Zur Gehaltsbestimmung werden die Integralflächen eines 1 H-NMR-Spektrums der Probe in Chloroform und eine GC-Analyse herangezogen. Der Gehalt an 2-(N-Methylanilino)-acrylnitril in der Lösung liegt nach 1 H-NMR bei 45,54 %. Die Ausbeute liegt somit bei 95,4 % bezogen auf das eingesetzte Methylanilin.

Die Behandlung der Cyanid-Abwässer erfolgt mit konz. H₂O₂ und NaOH 30 % bei pH 10-12 bis zu einem Restgehalt an Cyanid unter 30 mg/kg (<30 ppm).

### B) 2,2-Dimethyl-4-oxo-5-phenyl-valeronitril

In die trockene, mit Schutzgas gespülte Apparatur (Stahlkessel 250 L) wird eine Lithiumdiisopropylamid-Lösung in THF/n-Hexan eingefüllt (LDA-Lösung 25,1 % w/w, ca. 2M, 80,7 kg, 188,7 Mol) und mittels Solekühlung unter Stickstoff auf - 15 bis -20 °C abgekühlt. Unter Kühlung wird Isobutyronitril (11,4 kg, 165 Mol) so zudosiert, dass die Innentemperatur -10 °C nicht übersteigt. Nach vollständiger Zugabe wird das Zulaufgefäß mit Toluol (2 kg) nachgespült (45 min.).

Die Reaktionsmischung wird bei Temperaturen zwischen -10 und -20 °C 55-65 min. nachgerührt. Anschließend wird die Toluollösung von 2-(N-Methylanilino)-acrylnitril (47,1 %, 52,8 kg, 157,2 Mol) so zudosiert, dass die Temperatur im Innern unter Solekühlung bei -20 °C nicht über -10 °C steigt (90 min.). Das Zulaufgefäß und die Zuleitungen werden mit Toluol (5,0 kg) nachgespült. Die rotbraune Reaktionsmischung wird bei - 10 bis -20 °C während 60-90 min. nachgerührt. Der Gehalt an Edukt (2-(N-Methylanilino)-acrylnitril) in einer gaschromatographischen Analyse liegt daraufhin unter 2%.

Bei abgeschalteter Kühlung wird beginnend bei -10 bis -20 °C Benzylchlorid (23,9 kg, 188,8 Mol) zudosiert wobei man die Innentemperatur auf 5 °C ansteigen lässt. Bei Überschreiten dieser Temperatur wird unter Wasserkühlung gearbeitet. Ist eine Innentemperatur von 15 °C erreicht, wird mit einer Heizrate von 20 °C/h auf eine Innentemperatur von 50 °C erwärmt, während weiter Benzylchlorid zudosiert wird. Der Zeitbedarf für die Zugabe liegt bei 2,5 h.

Das Reaktionsgemisch wird während 3-4 h bei 50-55 °C gehalten, der Gehalt 2,2-Dimethyl-4-(N-methylanilino)-glutarsäuredinitril in einer gaschromatographischen Probe liegt dann unter 2 %.

Der Ansatz wird dann auf unter 25 °C abgekühlt und in einen Kessel überführt, in dem ein Drei-Phasen-Gemisch aus Eis (22,6 kg), Wasser (45,2 kg) und Toluol (22,6 kg) vorgelegt ist (10 min.). Zum Nachspülen wird Toluol (14 kg) verwendet. Diese Toluol/Wasser-Phasenmischung wird anschließend auf 35-40 °C erwärmt, und man lässt die Phasen trennen. Man entfernt den klaren Unterstand (Wasserphase, 75 kg) und belässt die Zwischenschicht bei der organischen Produktphase.

Zur organischen Phase werden zunächst Benzyltriethylammoniumchlorid (3,4 kg) und Eis (34,7 kg) und anschließend bei 0-15 °C innerhalb 10 min. Bromwasserstoffsäure (48%, 69,4 kg, 411,6 Mol) gegeben. Die Temperatur im Ansatz erhöht sich dadurch auf ca. 50 °C und Blausäure wird abgetrieben, die in einem mit Natronlauge (32 %) gefüllten Absorber zurückgehalten wird. Nach 6 h Rühren bei 50-60 °C wird dem rotbraunen Reaktionsgemisch eine Probe entnommen. Der Gehalt an 4-Benzyl-2,2-dimethyl-4-(N-methylanilino)-glutarsäuredinitril in der Mischung sollte nach GC-Analyse (GC = Gaschromatographie) unter 0,5% liegen.

Ist diese Bedingung erfüllt, lässt man die Phasen bei einer Innentemperatur unter 60 °C für 10-15 min. absitzen und überführt die Cyanwasserstoff- haltige, HBr-saure dunkle Wasserphase (Cyanid-Abwasser I, 90-110 kg) in einen dicht verschlossenen Tank. Die ebenso dunkel gefärbte organische Phase wird unter 30 °C gekühlt und dann mit einer Mischung aus Wasser (22,5 kg) und Natronlauge (30 %, 2,5 kg) während 5-10 min. bei 15-25 °C ausgerührt. Man lässt die deutlich heller gefärbte alkalische Wasserphase (pH 10-14) absitzen und lässt sie zur Nachbehandlung in einen Tank ab (Cyanid-Abwasser 2,25 kg). Danach wird die organische Phase mit Wasser (25 kg) bei 15-25 °C für 10-15 min. durchgerührt, und die nach 10-15 min. vollständig abgesetzte Wasserphase zum alkalischen Cyanid-Abwasser 2 (25 kg) abgetrennt. Der pH-Wert dieser Waschflüssigkeit sollte bei 7-9 liegen.

Die Toluolphase wird in eine Destillationsapparatur transferiert, Kessel und Zulaufverbindungen werden mit Toluol (5 kg) nachgespült. Das Toluol wird im Vakuum bis maximal 50 °C vollständig abdestilliert (Destillat 1a, 110-120 kg). Der Rückstand der Destillation wird in Isopropanol (22,7 kg) aufgenommen und anschließend im Vakuum das Solvens bis zu einer maximalen Innentemperatur von 60 °C vollständig abdestilliert (Destillat 1b, 23 kg). Die Azeotrop-Destillation mit Isopropanol (22,7 kg) wird nochmals in gleicher Weise wiederholt (Destillat 1c, 23 kg).

Der Rückstand der Azeotrop-Destillation wird bei 25-30 °C mit Isopropanol (16 kg) aufgenommen und zu einer Mischung aus Isopropanol (8,0 kg) und Heptan (26 kg) zudosiert, der zur Kristallisationssteuerung Kristallkeime des 2,2-Dimethyl-4-oxo-5-phenyl-valeronitrils (0,05 kg) zugegeben sind. Zulaufgefäß und Verbindungsleitungen werden mit Isopropanol (2,0 kg) nachgespült. Man kühlt die Kristallsuspension aus - 15 °C bis -20 °C ab und rührt mind. noch 2 h, aber höchstens 16 h nach. Die Kristallmasse wird abgesaugt und in einer vorgekühlten Mischung aus Isopropanol (8 kg) und Heptan (8 kg) bei -15 bis -20 °C mehrere min. resuspendiert und erneut angesaugt. Man erhält als feuchte Rohware 26,7 kg des 2,2-Dimethyl-4-oxo-5-phenyl-valeronitrils, neben insgesamt 66,9 kg Mutterlauge. Die Kristalle werden im Vakuum bei 30-35 °C getrocknet, nach Trocknung verbleiben 22,3 kg (70,6 %) eines Produktes mit einer Reinheit größer 90 % nach GC- Analyse.

### C) Reinigung des 2,2-Dimethyl-4-oxo-5-phenyl-valeronitrils

In einem 250-I-Emaille-Reaktor wird 2,2-Dimethyl-4-oxo-5-phenyl-valeronitril (85-90 %, 22,3 kg, 110,8 Mol) in einem Gemisch aus Isopropanol (40,0 kg) und Toluol (4,4 kg) suspendiert und durch Erwärmen dieser Mischung auf 50-55 °C unter Rühren vollständig in Lösung gebracht. Die danach auf 25-30 °C gekühlte Lösung wird auf eine mit Isopropanol (5 kg) befüllte Rührdrucknutsche gefüllt, die Lösung wird mit kristallinem 2,2-Dimethyl-4-oxo-5-phenyl-valeronitrils (0,05 kg) angeimpft und dann langsam auf 5-10 °C abgekühlt. Es wird solange gerührt, bis eine dicke Kristallsuspension gebildet ist. Dann wird bis auf -15 bits -20 °C abgekühlt und mind. 2 h oder über Nacht bei dieser Temperatur gerührt.

Das Produkt wird abgenutscht und zweimal mit auf -15 bis -20 °C vorgekühlte Isopropanol (je 4,8 kg) nachgewaschen. Die feuchte Kristallmasse (26,6 kg) wird im Vakuum bei 30-35 °C getrocknet, man erhält 16,6 kg Produkt (74,4 % Ausbeute) mit einer Reinheit von 96,1 % (GC- Analyse). Die Mutterlauge (53,2 kg) wird verworfen.

### D) 2-Benzyl-4,4-dimethyl-I-pyrrolin

Raney-Nickel (7,7 kg), das zuvor vom wässrigen Überstand durch Dekantieren befreit wurde, wird in einem 250-I-Stahlautoklaven mit Stickstoffgas überschichtet und dann in Methanol (67 kg) 15 min. suspendiert. Nach Abschalten der Rührung lässt man das Raney-Nickel 15-30 min. absitzen und drückt mit Stickstoff über ein Tauchrohr den Methanolüberstand durch ein mit Dicalite® belegtes Druckfilter ab. Der Katalysator wird mit der Lösung von 2,2-Dimethyl-4-oxo-5-phenyl-valeronitril (13,2 kg) in Toluol (92,4 kg) bei 15-20 °C überschichtet und mit Methanol (14,3 kg), das zum Nachspülen des Zugabebehälters der Toluollösung diente, versetzt. Die Apparatur wird dreimal mit Stickstoff bis 3 bar gefüllt und entspannt, um Luftsauerstoffzu verdrängen. Anschließend wird dreimal mit Wasserstoff bei **I** bar gespült und schließlich wird der Wasserstoffdruck auf 4,5-5,5 bar erhöht. Die Hydrierung wird bei 5,0 bar und 55-60 °C durch Einschalten der Rührung gestartet. Die Wasserstoffaufnahme kommt nach ca. 3 h zum Erliegen, in dieser Zeit sind 3,3 m³ Wasserstoff aufgenommen. Das Reaktionsgemisch wird auf 15-20 °C abgekühlt, die Führung ausgeschaltet und der Wasserstoffüberdruck entspannt. Die Apparatur wird 4 mal mit Stickstoff gespült und zur Reaktionskontrolle wird eine Probe entnommen. Die Summe aus nicht umgesetztem Edukt und überhydriertem Nebenprodukt sollte 10 % nicht übersteigen. Zeigt die Probe das geforderte Ergebnis, wird die Reaktionslösung über ein mit Dicalite® (0,5 kg) belegtes Druckfilter klar filtriert. Apparatur und Filterrückstand werden mit Methanol (1.0 kg) nachgespült und anschließend wird aus der Reaktionslösung das Methanol bei einer Innentemperatur von 75-80 °C abdestilliert. Der Rückstand der Destillation wird auf 20-30 °C abgefühlt und mit Wasser (49.5 kg) gewaschen. Die Zwei-Phasenmischung wird 5-10 min. durchgerührt, 20-30 min. zur Phasentrennung stehen gelassen und dann die Wasserphase (47-51 kg) entfernt. Bei 15-20 °C werden zur organischen Phase Eis (44 kg) und Wasser (44 kg) und anschließend konzentrierte Salzsäure (32 %, 17,7 kg) zugegeben und 5-10 min. gerührt. Die HCl-saure Wasserphase hat einen pH-Wert von 1-2. Man lässt die beiden Phasen absitzen (10-20 min.) und trennt die wässrige Pyrrolin-Extraktionsphase ab. Zu dieser HCl-sauren wässrigen Produktphase werden Marmite®, Wasser, mit dem die Ableitung nachgespült wurde (5,6 kg) und Toluol (86,9 kg) zugegeben. Unter Kühlung werden bei max. 25 °C Ammoniak-Lösung (24 %, 17,7 kg) zugesetzt. Der pH-Wert in der Wasserphase des Phasengemisches sollte bei 9-11 liegen. Das Zweiphasengemisch wird 5-10 min. nachgerührt. Danach lässt man die Phasen absitzen und trennt die Wasserphase ab. Die Toluolphase wird unter Nachspülen mit Toluol (5,5 kg) in eine Destillationsapparatur überführt und das Toluol wird unter Vakuum bei einer 50°C nicht übersteigenden Innentemperatur vollständig abdestilliert. Das gewonnene Toluoldestillat kann für Extraktionen wiederverwendet werden. Der Gehalt an Pyrrolin wird in einem Aliquot der Toluolphase (50 g) bestimmt, von dem zunächst der Trockenrückstand durch vollständiges Abdampfen des Toluols im Vakuum bestimmt wird. Dieser Trockenrückstand weist einen Gehalt von 70 % an gesuchtem 2-Benzyl-4,4-dimethyl-1-pyrrolin nach GC auf.

Aus 100,7 kg Produktlösung wird bei einem Trockengewichtsanteil von 13,74 % in der 50 g Probe und einem GC-Gehalt von 74,1% eine Ausbeute von 54,7 Mol 2-Benzyl-4,4-dimethyl-1-pyrrolin errechnet. Bezogen auf eingesetztes Oxovaleronitril ergibt sich eine Ausbeute von 84 %.

### E) 6-(4-Chlorphenyl)-2,2-dimethyl-7-phenyl-2,3-dihydro-1H-pyrrolizin

Zur direkt anschließenden Ringschlusssynthese des Pyrrolizins werden ω-Brom-4-chloracetophenon in 10 mol% Überschuss (60,2 Mol) und Natriumhydrogencarbonat in 36 mol% Überschuss (74,4 Mol) zum bestimmten Pyrrolin (54,7 Mol) eingesetzt.

Der Destillationsrückstand aus Stufe D wird bei 15-20 °C mit Methanol (49 kg) danach mit Natriumhydrogencarbonat (6,25 kg) und zuletzt unter Kühlung mit ω-Brom-4-chloracetophenon (14,06 kg) versetzt. Die resultierende hellgelbe, dünnflüssige Suspension wird unter Lichtausschluss während 17-20 h bei 18-25 °C gerührt. Die Suspension wird zentrifugiert und das Zentrifugat mit Methanol (11 kg) in zwei Portionen nachgewaschen.

Methanolmutterlauge und Methanolwaschlösungen werden entsorgt. Es werden 16,5-18,5 kg feuchtes Rohprodukt erhalten, das in Wasser (88 kg) angeschlämmt und bei 40-45 °C 1-2 h gerührt wird. Das von mineralischen Begleitsubstanzen gereinigte Rohprodukt wird abzentrifugiert und mit Wasser (22 kg) in 2 Portionen gewaschen. Die Ausbeute an feuchten Rohprodukten beträgt 14-16 kg. Die wässrige Mutterlauge und wässrige Waschphasen werden verworfen.

Das Rohprodukt wird im Vakuum bei 35-40 °C getrocknet. Beim Trocknen reduziert sich die Gewichtsmenge auf 12,5-13,5 kg (38,4 Mol -41,95 Mol) 6-(4-Chlorphenyl)-2,2-dimethyl-7-phenyl-2,3-dihydro-1H-pyrrolizin vom Gehalt 97.3 % (HPLC). Dies entspricht einer Ausbeute von 71,0-76,7 % bezogen auf das in der Hydrierung gewonnene Pyrrolin und einer Ausbeute von 59-64 % bezogen auf das in der Hydrierung eingesetzte Oxo-valeronitril. Der Gehalt an isomerem 5-(4-Chlorphenyl)-2,2-dimethyl-7-phenyl- 2,3-dihydro-1H-pyrrolizin liegt unter 2 %, der Gehalt an ω-Brom-4-chloracetophenon unter 0,1 % und der Gehalt an mineralischen Begleitsubstanzen unter 0,5 % (Aschebestimmung).

### Beispiel 2

Die Stufen A) bis C) wurden wie in Beispiel I durchgeführt.

### E) 2-Benzyl-2-(2-cyano-2-methyl_propyl)-1,3-dioxolan

Oxovaleronitril (50 g, 0,25 Mol) wird mit Ethylenglykol (75 g, 1,21 Mol) und p-Toluolsulfonsäure (9,2 g, 0,048 Mol) in Toluol (300 ml, 260,1 g, 2.82 Mol) versetzt, und die Reaktionsmischung wird langsam bis zum Sieden aufgeheizt (2,5 h). Nach weiteren 2 h Reflux wird der Ansatz mittels GC überprüft. Das Toluol wird während der Anheiz- und Reflux phase abdestilliert und durch trockenes Solvens (185,3 g) ersetzt. Der Ansatz wird bis zur Aufarbeitung unter trockenem N₂ kaltgestellt. Zur Aufarbeitung wird die Toluollösung des Rohproduktes mit eiskalter Natronlauge (25 g, 0,625 Mol NaOH auf 150 g Eis) extrahiert und die Phasen werden getrennt. Die organische Phase wird mit wasserfreiem Magnesiumsulfat (MG 120,37, 50 g, 0,4 Mol) getrocknet. Nach Filtration erhält man 245 g Filtrat.

### F) 2-Benzyl-4,4-dimethyl-1-pyrrolin

Die unter E) gewonnene rohe Lösung des Dioxolans wird in einem 1L-Autoklaven vorgelegt und dann werden 20 g Raney-Nickel B113W (MG 58,71, 0,34 Mol), das zuvor dreimal mit wasserfreiem Methanol ausgelaugt wurde, zusammen mit 71,1g Toluol zugegeben. Durch dreimaliges Aufpressen von Stickstoff und nachfolgendes Entspannen wird Luftsauerstoff aus dem Autoklaven verdrängt. Die Hydrierung startet, nachdem mit dreimal aufeinanderfolgenden Wasserstoffaufgaben und Entlüftung ein Hydrierdruck von 48 bar aufgegeben wurde und die Manteltemperatur des Autoklaven auf 63°C eingestellt wurde (Zeitbedarf 3 h). Die Hydrierung im 1L-Autoklaven erfordert die Nachbefüllung mit Wasserstoff auf den Ausgangsdruckwert nach ca 3 h (Innendruck 23 bar) und nach weiteren 18 h (Innendruck 17 bar). Nach einer Hydrierdauer von insgesamt 26,5 h lässt man abkühlen und filtriert das Reaktionsprodukt über Decalit.

Die Spaltung des Acetals erfolgt direkt im Anschluss, indem das Rohprodukt in verdünnter Salzsäure (HCl 32 %, 50 g, 0,43 Mol in H₂O, 200 g) aufgenommen und 1 h bei 30 °C gerührt wird. Man zieht den organischen Überstand (Toluolphase) ab und alkalisiert bei 0 bis 5 °C die Wasserphase mit wässrigem konzentriertem Ammoniak (25 %, 50 g, 0,73 Mol) auf einen pH-Wert von 9 bis 10. Das abgeschiedene Pyrrolin wird in Diethylether (200 g) aufgenommen und abgetrennt. Nach Verdampfen des Ethers im Vakuum verbleiben 32,1 g Produkt. Das 2-Benzyl-4,4-dimethyl-I-pyrrolin wird in einer Ausbeute von 69 % und einer Reinheit von 92,6 % (GC) erhalten.

Wird das Dioxolan vor der Verwendung in der Hydrierung durch Destillation gereinigt (92 %, GC), wird bei der Hydrierung eine höhere Hydriergeschwindigkeit bei niedrigeren Drucken (5 bar) und niedrigeren Temperaturen erreicht. Die Reinheit des erhaltenen Pyrrolins beträgt 94-98 % (GC).

### Beispiel 3

### Herstellung von ML 3000:

### A) 5-(4-Chlorphenyl)-2,2-dimethyl-7-phenyl-2,3-dihydro-1H-pyrrolizin

17,9 kg (95,5 Mol) des gemäß Beispiel 1 oder 2 hergestellten 2-Benzyl-4,4-dimethyl-1-pyrrolins (bezogen auf Gehalt an Pyrrolinverbindung), 29,7 kg (127,2 Mol, 1,33 Äquiv.) o-Brom-4-chloracetophenon und 226,6 kg Methanol werden in einem Reaktor (500 1) vorgelegt. Nach Zugabe von 12,7 kg (151,2 Mol, 1,58 Äquiv.) Natriumhydrogencarbonat wird unter Lichtausschluss bei 17-24 °C unter Bildung einer beigen Suspension gerührt. Die Reaktion wird weitergeführt, bis der Restgehalt an Pyrrolinverbindung in der Mischung < 5 % beträgt. Nach 17 h wird eine Probe gezogen und mittels Gaschromatographie auf Gehalt an Pyrrolinverbindung geprüft. Die Analyse ergab einen Gehalt von 2 %. Danach wird die Suspension bei einer Innentemperatur von 18-22 °C zentrifugiert und der durch Zentrifugation erhaltene Feststoff wird mit 14,4 kg Methanol in zwei Portionen ausgewaschen. Das noch feuchte, schwach gelbe Produkt wiegt 25,8 kg.

Das noch feuchte Rohprodukt (25,8 kg) wird in 150 kg Wasser suspendiert, dann innerhalb 15 min auf eine Innentemperatur von 50-60 °C erwärmt und 40 min bei dieser Temperatur gerührt Die auf 40 °C abgekühlte Suspension (40 min) wird zentrifugiert und der durch Zentrifugieren erhaltene hellgelbe, kristalline Feststoff wird mit 27 kg Wasser in zwei Portionen ausgewaschen. Das Produkt wird im Vakuum bei 50-60 °C während 12-24 h getrockent. Man erhält 18,6 kg 6-(4-Chlorphenyl)-2,2-dimethyl-7-phenyl-2,3-dihydro-1H-pyrrolizin, mit einem Gehalt an 0,33 % Asche und einem Isomerengehalt an 5-(4-Chlorphenyl)-2,2-dimethyl-7-phenyl-2,3-dihydro-1H-pyrrolizin von 1,0 %.

### B) 6-(4-Chlorphenyl)-2,2-dimethy)-7-phenyl-2,3-dihydro-1H-pyrrolizin-5-yl-essigsäure (ML-3000)

In einem 250 1-Reaktor werden, nach dreimaligem Evakuieren und N₂-Einleiten, 11,5 kg (35,7 Mol) 6-(4-Chlorphenyl)2,2-dimethyl-7-phenyl-2,3-dihydro-1H-pyrrolizin in 60 kg Tetrahydrofuran (THF) vorgelegt. Unter 0,5 bar Stickstoffzufluss (N₂) wird die gelbgefarbte Lösung 10- 15 °C abgekühlt. Anschließend werden unter N₂ 6,8 kg (54,7) Mol Oxalylchlorid aus einem Zulaufgefäß über 35 min so zudosiert, dass die Innentemperatur 20 °C nicht überschreitet.

Nach beendeter Zugabe wird die nun dunkelgrüne, dünne Suspension 20 bis 30 min bei einer Innentemperatur von 18-25 °C nachgerührt.

In einem 500 1-Reaktor werden 18 kg Eis in Schuppen vorgelegt. Zu diesem Eis wird die 25 °C warme Suspension über 5 min so zudosiert, dass die Innentemperatur des Gemisches 20 °C nicht überschreitet.

Das Reaktionsgemisch wird noch 10-20 min bei einer Innentemperatur von 25-35 °C gerührt. Die noch immer grüne Lösung wird bei 25-35 °C mit 62,2 kg Diethylenglykol verdünnt. Dann werden aus einem Zulaufgefäß unter Kühlung 14,9 kg (298 Mol) Hydrazinhydrat während 10-15 min zugegeben. Die Innentemperatur steigt auf maximal 40-45 °C an. Durch schrittweises Erhöhen der Temperatur während 1,5 h wird die inzwischen beige gefärbte Suspension auf eine Innentemperatur von 70-75 °C erwärmt, wobei THF abdestilliert wird. Bis zum Erreichen einer Innentemperatur von 75°C werden 45,4 kg THF-Destillat gesammelt.

Das Reaktionsgemisch wird auf 50-55 °C abgekühlt und in 8 bis 10 Portionen über 45 min verteilt mit insgesamt 26,4 kg Kaliumhydroxid in Schuppen (KOH) versetzt, wobei die Innentemperatur bereits bei den ersten 5 kg KOH auf 65-70 °C ansteigt und die anfangs dicke Suspension sich gelb verfärbt, dünnflüssiger wird und kurzfristig leichter Rückfluss auftritt.

Diese Suspension wird nun mit einem. Temperaturanstieg von 15 °C/h auf 90 °C erwärmt, wobei ab 85 °C ein leichtes Schäumen einsetzt und die Suspension eindickt. Mit einer Temperatursteigerung von 2 °C/h wird die Innentemperatur nun weiter auf 102 °C angehoben und gleichzeitig bei erhöhter Rührerdrehzahl durch das Tauchrohr Stickstoff durch die Reaktionsmischung geblasen. Durch starkes Aufschäumen und zusätzliche Gasentwicklung nimmt das Volumen des Reaktorinhaltes auf das Doppelte zu. Bei Bedarf wird die Reaktionstemperatur durch Kühlung abgesenkt. Bei einer Innentemperatur von 100-105 °C beginnt der Schaum zusammenzufallen und eine rotbraune dünne Suspension entsteht, die nun mit einer Heizgeschwindigkeit von 15 °C/h weiter auf eine Innentemperatur von 140-145 °C. aufgeheizt wird. Bei übermäßigem Schäumen wird die Reaktionstemperatur durch Kühlung kurzzeitig abgesenkt. Gleichzeitig werden mehrer wässrige Destillate von insgesamt 44 kg gesammelt.

Der Ansatz wird 2-2,5 h bei 120-145 °C gehalten. Danach wird die Reaktortemperatur auf 30-40 °C abgekühlt und es werden 74,7 kg Wasser und 56,7 kg Diethylether zugegeben. Die Reaktionsmischung wird 10-15 min bei einer Innentemperatur von 30-33 °C durchgerührt, dann lässt man die Phasen absitzen. Das entstehende Dreiphasensystem wird getrennt. Die unterste stark alkalische wässrige Phase, die 154,9 kg wiegt, ist farblos und nur schwach getrübt. Sie wird als Abwasser entsorgt. Die gelbgefärbte, trübe Zwischenphase von öliger Konsistenz wiegt 29,6 kg und enthält die Hauptmenge an Produkt als Kaliumsalz. Die oberste, klare, gelbgefärbte etherische Phase wird bei einer Innentemperatur von 30 °C in einer Extraktionsapparatur mit 10 kg Wasser 10 min kräftig gerührt. 10 min nach Abstellen der Rührung wird die Wasserphase abgetrennt. Die Zwischenphase (29,6 kg) und der wässrige Extrakt der Etherphase (10,9 kg) werden in einer Extraktionsapparatur mit 126,2 kg Diethylether und 59,7 kg Wasser versetzt, und das Gemisch wird auf eine Innentemperatur von 0-5 °C abgekühlt.

Über ein Zulaufgefäß wird nun ein Gemisch aus 6,0 kg 32,5%-iger Salzsäure und 6,0 kg Wasser während 15 min so zudosiert, dass eine maximale Innentemperatur von 10 °C nicht überschritten und ein pH-Wert von 1-2 erreicht wird. Ist dieser pH-Wert nicht erreicht, werden weitere 0,2 kg 32,5%-iger Salzsäure im Gemisch mit 0,2 kg Wasser zugegeben. Nach Erreichen dieses pH-Wertes werden die Phasen noch 5-10 min gut durchgerührt und dann zur Phasentrennung bei abgeschaltetes Rührung 10-20 min stehen gelassen.

Die HCl-saure Wasserphase wird abgelassen. Die Etherphase wird nochmals über das Zulaufgefäß mit einer Mischung aus 9,5 kg Salzsäure und 19 kg Wasser versetzt und bei einer 10 °C nicht überschreitenden Innentemperatur 5-10 min gut durchgerührt. Die Phasen werden getrennt und die HCl-Behandlung wird gewünschtenfalls bis zu 3 Mal wiederholt.

Die Etherphase wird danach mit 30 kg demineralisiertem Wasser versetzt, während 10-20 min gut durchgerührt und auf 15-20 °C erwärmt. Man trennt die Phasen und wiederholt die Extraktion.

Die von Säurespuren freigewaschene Etherphase wird mit 6,5 kg wasserfreiem Magnesiumsulfat und 0,4 kg Aktivkohle (Acticarbon 2S), die in 1 kg Diethylether angeschlämmt werden, versetzt und 30-45 min bei 18 °C gerührt. Die Suspension wird über ein mit 0,5 kg Filtrierhilfsmittel (Cell flock) belegtes Druckfilter in eine Destillationsapparatur klarfiltriert. Filter und Apparatur werden mit 8 kg Diethylether nachgespült.

Zur Etherphase werden 95,6 kg Heptan gegeben, und bei einer Innentemperatur von 15-20 °C wird der Ether unter Vakuum abdestilliert. Die nach Abdestillieren des Ethers entstandene Kristallsuspension wird auf eine Innentemperatur von 13-18 °C gekühlt und 0,5-1,5 h bei dieser Temperatur gerührt. Anschließend werden die Kristalle abzentrifugiert. Das gewonnene feuchte Produkt wird mit 23,0 kg Heptan in 2 Portionen gewaschen, Das feuchte Produkt wird bei 50-60 °C über Nacht im Vakuumtrockenschrank getrocknet und gewünschtenfalls vermahlen. Man erhält 10,5 kg (77,2 %) ML-3000 mit einem Schmelzpunkt, bestimmt nach der DSC-Methode, von 157 °C. Das IR-Spektrum entspricht dem des Referenzstandards.

## Patentansprüche

1. Verfahren zur Herstellung der Verbindung der Formel I 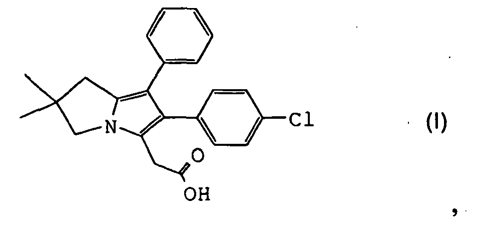 wobei man
a) die Verbindung der Formel IV 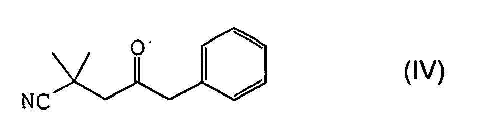 in die Verbindung der Formel III 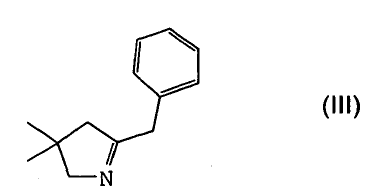 überführt, indem man
a1) die Verbindung der Formel IV in einer Reinheit von mehr als 90% (m/m) einsetzt und katalytisch mit wasserfreiem Raney-Nickel in Toluol oder einem Gemisch aus Toluol und einem C₁-C₄- Alkohol hydriert oder
a2) die Verbindung der Formel IV in das Ketal der Formel IVa 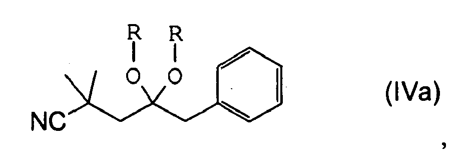 worin die Reste R, die gleich oder verschieden sein können, für C₁-C₄-Alkyl oder zusammen für C₂-C₃-Alkylen stehen, überführt und das Ketal mit wasserfreiem Raney-Nickel bei 5-50 bar Wasserstoffdruck in einem alkoholischen oder aromatischen Lösungsmittel bei einer Temperatur im Bereich von Raumtemperatur bis 70°C katalytisch hydriert,
b) die Verbindung der Formel III mit ω-Brom-4-chloracetophenon zu einer Ver bindung der Formel II 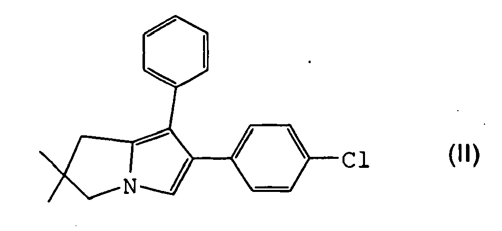 umsetzt und
c) in die Verbindung der Formel II einen Essigsäurerest einführt.

2. Verfahren zur Herstellung der Verbindung der Formel II 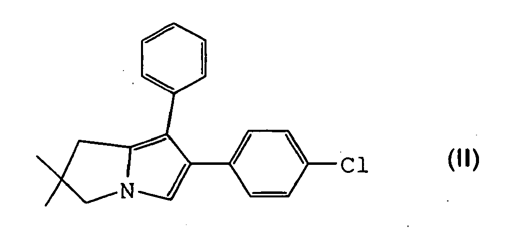 wobei man die Verbindung der Formel IV 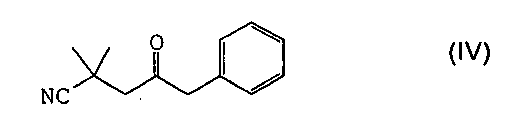 in die Verbindung der Formel III 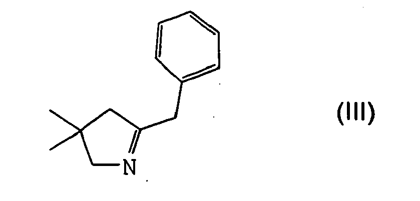 überführt, indem man
a1) die Verbindung der Formel IV in einer Reinheit von mehr als 90% (m/m) einsetzt und katalytisch mit wasserfreiem Raney-Nickel in Toluol oder einem Gemisch aus Toluol und einem C₁-C₄Alkohol hydriert, oder
a2) die Verbindung der Formel IV in das Ketal der Formel IVa 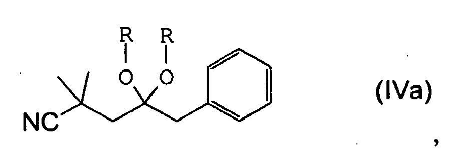 worin die Reste R, die gleich oder verschieden sein können, für C₁-C₄-Alkyl oder zusammen für C₂-C₃-Alkylen stehen, überführt und das Ketal mit wasserfreiem Raney-Nickel bei 5-50 bar Wasserstoffdruck in einem alkoholischen oder aromatischen Lösungsmittel bei einer Temperatur im Bereich von Raumtemperatur bis 70°C katalytisch hydriert, und
b) die Verbindung der Formel III mit ω-Brom-4-chloracetophenon zu einer Verbindung der Formel II umsetzt.

3. Verfahren zur Herstellung der Verbindung der Formel III 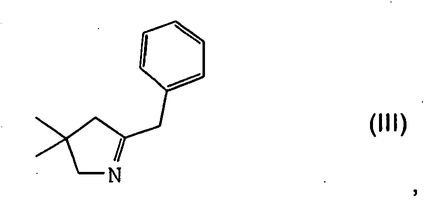 wobei man die Verbindung der Formel IV 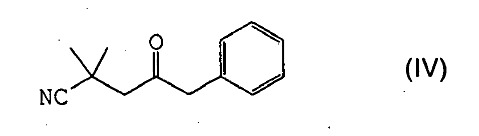 durch katalytische Hydrierung in die Verbindung der Formel III überführt, indem man
a1) die Verbindung der Formel IV in einer Reinheit von mehr als 90% (m/m) einsetzt und katalytisch mit wasserfreiem Raney-Nickel in Toluol oder einem Gemisch aus Toluol und einem C₁-C₄Alkohol hydriert, oder
a2) die Verbindung der Formel IV in das Ketal der Formel IVa 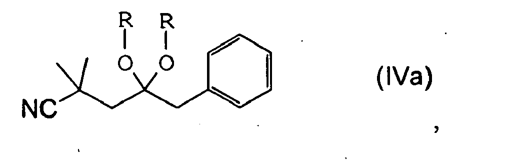 worin die Reste R, die gleich oder verschieden sein können, für C₁-C₄-Alkyl oder zusammen für C₂-C₃-Alkylen stehen, überführt und das Ketal mit wasserfreiem Raney-Nickel bei 5-50 bar Wasserstoffdruck in einem alkoholischen oder aromatischen Lösungsmittel bei einer Temperatur im Bereich von Raumtemperatur bis 70°C katalytisch hydriert.

4. Verfahren nach einem der vorhergehenden Ansprüche, **dadurch gekennzeichnet, dass** man die Verbindung der Formel IV in einer Reinheit von mindestens 95 % einsetzt.

5. Verfahren nach einem der vorhergehenden Ansprüche, **dadurch gekennzeichnet, dass** man die Verbindung der Formel IV erhält durch Michaeladdition von Isobutyronitril an eine Verbindung der Formel V 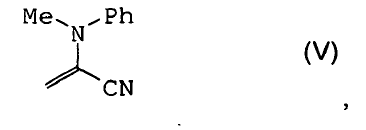 Benzylierung des Michael-Additionsproduktes zu 2-Benzyl-4,4-dimethyl-2-(N-methylanilino)glutaronitril und Hydrolyse dieses Nitrils.

6. Verfahren nach Anspruch 5, **dadurch gekennzeichnet, dass** das Isobutyronitril mit Lithiumdiisopropylamid in Toluol deprotoniert wird.

7. Verfahren nach Anspruch 5 oder 6, **dadurch gekennzeichnet, dass** die Reaktionstemperatur bei der Michael-Addition im Bereich von etwa -10 °C bis -20 °C liegt.

8. Verfahren nach einem der Ansprüche 5 bis 7, **dadurch gekennzeichnet, dass** die Hydrolyse des Nitrils im Sauren in einem Zweiphasensystem unter Phasentransferkatalyse erfolgt.

9. Verfahren nach einem der Ansprüche 5 bis 8, **dadurch gekennzeichnet, dass** man die Verbindung der Formel V erhält durch Umsetzung von Chloracetaldehyd, N-Methylanilin und einem Alkalimetallcyanid und anschließende basische Eliminierung.

10. Verfahren nach Anspruch 9, **dadurch gekennzeichnet, dass** man Chloracetaldehyd und anschließend das Alkalimetallcyanid zu N-Methylanilin gibt.

11. Verfahren nach Anspruch 9 oder 10, **dadurch gekennzeichnet, dass** man Chloracetaldehyd, N-Methylanilin und das Alkalimetallcyanid im Molverhältnis von etwa 1,1 bis 1,3:1:1,1 bis 1,3 einsetzt.

12. Verfahren nach einem der Ansprüche 9 bis 11, **dadurch gekennzeichnet, dass** man die basische Eliminierung im Zweiphasensystem unter Phasentransferkatalyse durchführt.

## Claims

1. Process for the preparation of the compound of the formula I, 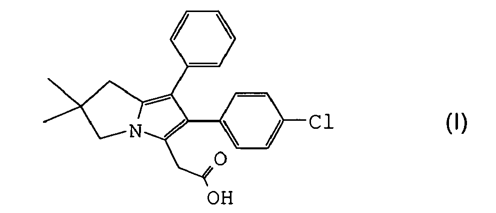 where
a) the compound of the formula IV 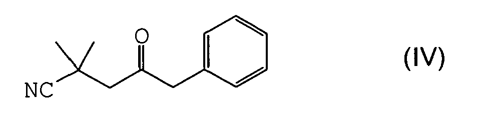 is converted into the compound of the formula III 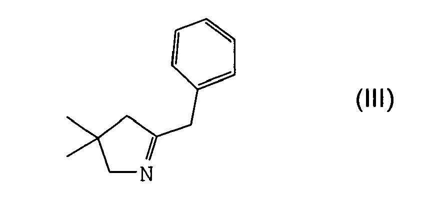 by
a1) employing the compound of the formula IV in a purity of more than 90% (m/m) and catalytically hydrogenating the compound using anhydrous Raney nickel in toluene or a mixture of toluene and a C₁-C₄-alcohol, or
a2) converting the compound of the formula IV into the ketal of the formula IVa 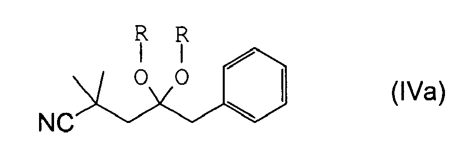 in which the radicals R, which can be identical or different, are C₁-C₄-alkyl or together are C₂-C₃-alkylene, and catalytically hydrogenating the ketal using anhydrous Raney nickel at 5-50 bar hydrogen pressure in an alcoholic or aromatic solvent at a temperature in the range from room temperature to 70°C,
b) reacting the compound of the formula III mit ω-bromo-4-chloroacetophenone to give a compound of the formula II 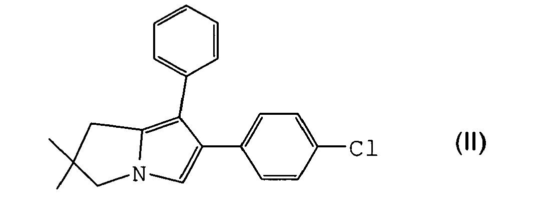 and
c) introducing an acetic acid radical into the compound of the formula II.

2. Process for the preparation of the compound of the formula II 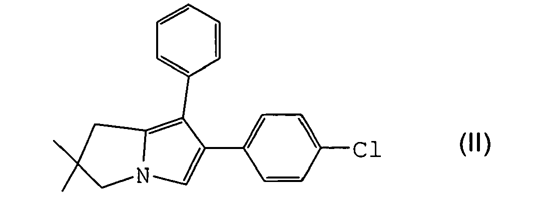 where the compound of the formula IV 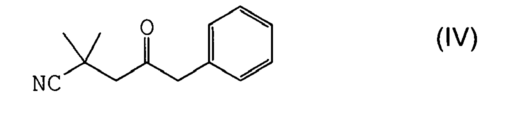 is converted into the compound of the formula III 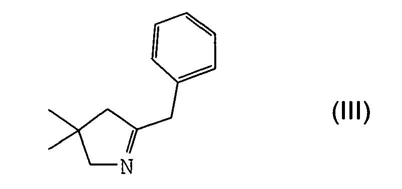 by
a1) employing the compound of the formula IV in a purity of more than 90% (m/m) and catalytically hydrogenating the compound using anhydrous Raney nickel in toluene or a mixture of toluene and a C₁-C₄-alcohol, or
a2) converting the compound using anhydrous Raney nickel in toluene or a mixture of toluene and a C₁-C₄-alcohol, into the ketal of the formula IVa 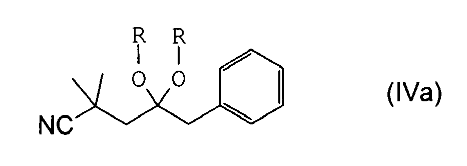 in which the radicals R, which can be identical or different, are C₁-C₄-alkyl or together are C₂-C₃-alkylene, and catalytically hydrogenating the ketal using anhydrous Raney nickel at 5-50 bar hydrogen pressure in an alcoholic or aromatic solvent at a temperature in the range from room temperature to 70°C, and
b) reacting the compound of the formula III with ω-bromo-4-chloroacetophenone to give a compound of the formula II.

3. Process for the preparation of the compound of the formula III 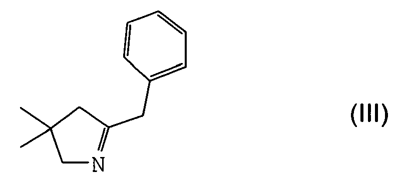 where the compound of the formula IV 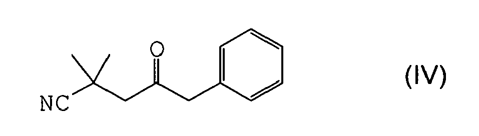 is converted by catalytic hydrogenation into the compound of the formula III by
a1) employing the compound of the formula IV in a purity of more than 90% (m/m) and catalytically hydrogenating the compound using anhydrous Raney nickel in toluene or a mixture of toluene and a C₁-C₄-alcohol, or
a2) converting the compound of the formula IV into the ketal of the formula IVa 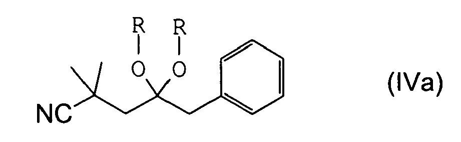 in which the radicals R, which can be identical or different, are C₁-C₄-alkyl or together are C₂-C₃-alkylene, and catalytically hydrogenating the ketal using anhydrous Raney nickel at 5-50 bar hydrogen pressure in an alcoholic or aromatic solvent at a temperature in the range from room temperature to 70°C.

4. Process according to one of the preceding claims, **characterized in that** the compound of the formula IV is employed in a purity of at least 95%.

5. Process according to one of the preceding claims, **characterized in that** the compound of the formula IV is obtained by Michael addition of isobutyronitrile to a compound of the formula V 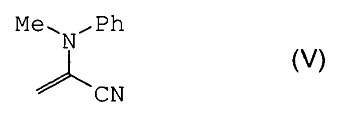 benzylation of the Michael addition product to give 2-benzyl-4,4-dimethyl-2-(N-methyl-anilino)glutaronitrile and hydrolysis of this nitrile.

6. Process according to Claim 5, **characterized in that** the isobutyronitrile is deprotonated using lithium diisopropylamide in toluene.

7. Process according to Claim 5 or 6, **characterized in that** the reaction temperature in the Michael addition is in the range of approximately -10°C bis -20°C.

8. Process according to Claims 5 to 7, **characterized in that** the hydrolysis of the nitrile in the acid is carried out in acidic medium in a two-phase system under phase-transfer catalysis.

9. Process according to Claims 5 to 8, **characterized in that** the compound of the formula V is obtained by reaction of chloroacetaldehyde, N-methylaniline and an alkali metal cyanide and subsequent basic elimination.

10. Process according to Claim 9, **characterized in that** chloroacetaldehyde and then the alkali metal cyanide are added to N-methylaniline.

11. Process according to Claim 9 or 10, **characterized in that** chloroacetaldehyde, N-methyl-aniline and the alkali metal cyanide are employed in a molar ratio of approximately 1.1 to 1.3:1:1.1 to 1.3.

12. Process according to Claims 9 to 11, **characterized in that** the basic elimination is carried out in the two-phase system under phase-transfer catalysis.

## Revendications

1. Procédé de production du composé de formule I, 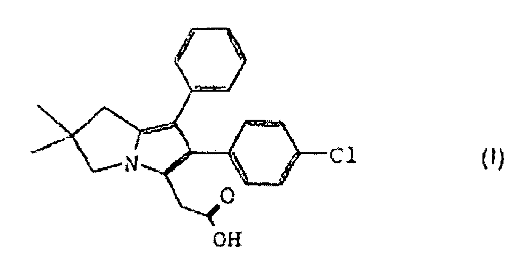 dans lequel on convertit :
a) le composé de formule IV : 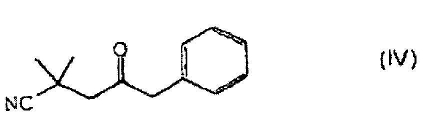 en le composé de formule III : 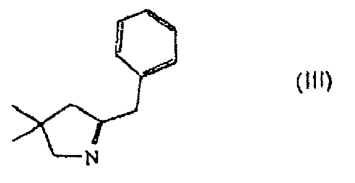 en
a1) utilisant le composé de formule IV ayant une pureté supérieure à 90 % (m/m) et en hydrogenant par voie catalytique avec du nickel de Raney anhydre dans du toluène ou un mélange de toluène et d'un alcool en C₁ à C₄, ou
a2) en convertissant le composé de formule IV en le cétal de formule IVa : 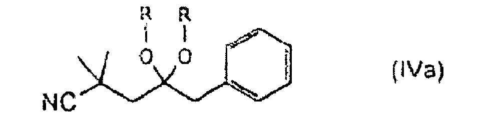 dans laquelle les radicaux R, qui peuvent être identiques ou différents, sont un groupe alkyle en C₁ à C₄ ou conjointement un groupe alkylène en C₂ à C₃, et en hydrogenant par voie catalytique le cétal avec du nickel de Raney anhydre à une pression d'hydrogène de 5 à 50 bars dans un solvant alcoolique ou aromatique à une température dans la gamme de la température ambiante à 70°C,
b) on convertit le composé de formule III avec de la ω-bromo-4-chloroacétophénone en un composé de formule II : 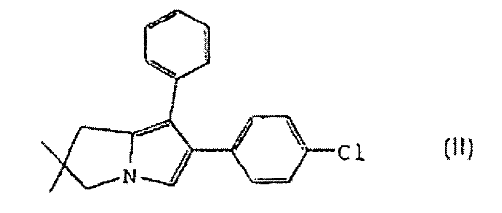 et
c) on introduit un radical acide acétique dans le composé de formule II.

2. Procédé de production du composé de formule II : 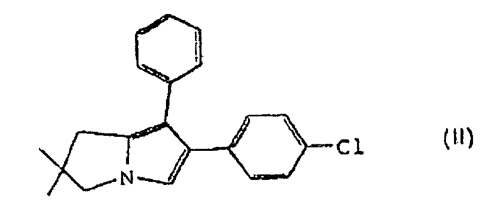 dans lequel on convertit le composé de formule IV : 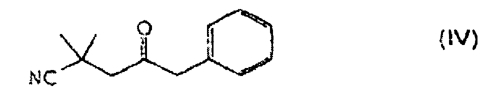 en le composé de formule III : 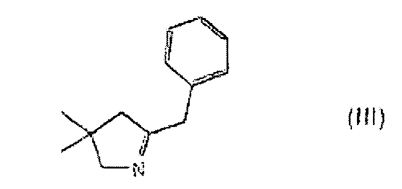 en
a1) utilisant le composé de formule IV ayant une pureté supérieure à 90 % (m/m) et en hydrogenant par voie catalytique avec du nickel de Raney anhydre dans du toluène ou un mélange de toluène et d'un alcool en C₁ à C₄, ou
a2) en convertissant le composé de formule IV en le cétal de formule IVa : 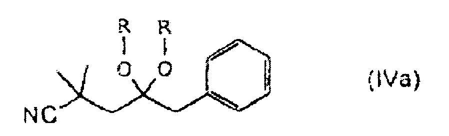 dans laquelle les radicaux R, qui peuvent être identiques ou différents, sont un groupe alkyle en C₁ à C₄ ou conjointement un groupe alkylène en C₂ à C₃, et en hydrogenant par voie catalytique le cétal avec du nickel de Raney anhydre à une pression d'hydrogène de 5 à 50 bars dans un solvant alcoolique ou aromatique à une température dans la plage de la température ambiante à 70 °C,
b) on convertit le composé de formule III avec de la ω-bromo-4-chloroacétophénone en un composé de formule II.

3. Procédé de production du composé de formule III : 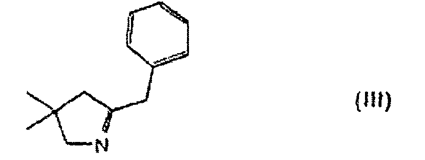 dans lequel on convertit le composé de formule IV : 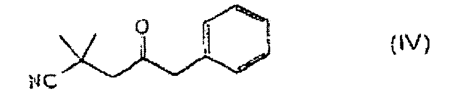 en le composé de formule III par hydratation catalytique, en
a1) utilisant le composé de formule IV ayant une pureté supérieure à 90 % (m/m) et en hydrogenant par voie catalytique avec du nickel de Raney anhydre dans du toluène ou un mélange de toluène et d'un alcool en C₁ à C₄, ou
a2) en convertissant le composé de formule IV en le cétal de formule IVa : 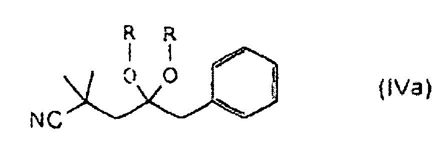 dans laquelle les radicaux R, qui peuvent être identiques ou différents, sont un groupe alkyle en C₁ à C₄ ou conjointement un groupe alkylène en C₂ à C₃, et en hydrogenant par voie catalytique le cétal avec du nickel de Raney anhydre à une pression d'hydrogène de 5 à 50 bars dans un solvant alcoolique ou aromatique à une température dans la plage de la température ambiante à 70 °C.

4. Procédé selon l'une quelconque des revendications précédentes, **caractérisé en ce que** l'on utilise le composé de formule IV avec une pureté d'au moins 95 %.

5. Procédé selon l'une quelconque des revendications précédentes, **caractérisé en ce que** l'on obtient le composé de formule IV par une addition de Michael à partir d'isobutyronitrile sur un composé de formule V : 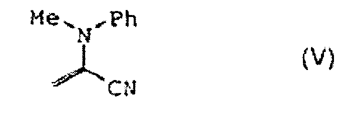 Benzylation du produit d'addition de Michael en 2-benzyl-4,4-diméthyl-2-(N-méthylanilino)glutaroni-trile et hydrolyse de ce nitrile.

6. Procédé selon la revendication 5, **caractérisé en ce que** l'isobutyronitrile est déprotoné avec du diisopropylamide de lithium dans du toluène.

7. Procédé selon la revendication 5 ou 6, **caractérisé en ce que** la température de réaction pour l'addition de Michael est dans une plage d'environ -10 °C à -20 °C.

8. Procédé selon l'une quelconque des revendications 5 à 7, **caractérisé en ce que** l'hydrolyse du nitrile s'effectue en milieu acide dans un système biphasique par catalyse de transfert de phase.

9. Procédé selon l'une quelconque des revendications 5 à 8, **caractérisé en ce que** l'on obtient le composé de formule V par conversion du chloroacétaldéhyde, de la N-méthylaniline et d'un cyanure de métal alcalin suivie d'une élimination en milieu basique.

10. Procédé selon la revendication 9, **caractérisé en ce que** l'on ajoute le chloroacétaldéhyde puis le cyanure de métal alcalin à la N-méthylaniline.

11. Procédé selon la revendication 9 ou 10, **caractérisé en ce que** l'on utilise le chloroacétaldéhyde, la N-méthylaniline et le cyanure de métal alcalin en un rapport molaire d'environ 1,1 à 1,3:1:1,1 à 1,3.

12. Procédé selon l'une quelconque des revendications 9 à 11, **caractérisé en ce que** l'on effectue l'élimination en milieu basique dans un système biphasique par catalyse de transfert de phase.
